(19) Europäisches Patentamt
European Patent Office
Office européen des brevets

(11) Veröffentlichungsnummer: **0 347 788 B1**

# (12) EUROPÄISCHE PATENTSCHRIFT

(45) Veröffentlichungstag der Patentschrift: **10.08.94**

(21) Anmeldenummer: **89111066.0**

(22) Anmeldetag: **19.06.89**

(51) Int. Cl.5: **C07D 239/52**, A01N 47/36, C07D 239/42, C07D 239/47, C07D 251/14, C07D 401/12, C07D 417/12, C07D 403/12, //C07D285/10,C07D207/48, C07C311/00

## (54) Substituierte Sulfonyldiamide, Verfahren zu ihrer Herstellung und ihre Verwendung als Herbizide und Pflanzenwachstumsregulatoren.

(30) Priorität: **18.06.88 DE 3820647**
**06.07.88 DE 3822841**

(43) Veröffentlichungstag der Anmeldung:
**27.12.89 Patentblatt 89/52**

(45) Bekanntmachung des Hinweises auf die Patenterteilung:
**10.08.94 Patentblatt 94/32**

(84) Benannte Vertragsstaaten:
**AT BE CH DE ES FR GB IT LI NL**

(56) Entgegenhaltungen:
**EP-A- 0 131 258**

**CHEMICAL ABSTRACTS, Band 68, 1968, Seite 3774, Zusammenfassung-Nr. 39000d, Columbus, Ohio, US; R. APPEL et al.: "Sulfimide. III. Derivatives of sulfamoylsulfamide"**

(73) Patentinhaber: **Hoechst Schering AgrEvo GmbH**
**Gerichtstrasse 27**
**D-13342 Berlin (DE)**

(72) Erfinder: **Willms, Lothar, Dr.**
**Schulstrasse 3**
**D-5416 Hillscheid (DE)**
Erfinder: **Bauer, Klaus, Dr.**
**Doorner Strasse 53D**
**D-6450 Hanau 7 (DE)**
Erfinder: **Bieringer, Hermann, Dr.**
**Eichenweg 26**
**D-6239 Eppstein/Taunus (DE)**
Erfinder: **Bürstell, Helmut, Dr.**
**Am Hohlacker 65**
**D-6000 Frankfurt am Main 50 (DE)**

**EP 0 347 788 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 82, 1975, Seite 446, Zusammenfassung-Nr. 30627v, Columbus, Ohio, US; H. YAMAGUCHI et al.: "Sulfonyl diamide derivatives. II. Amide exchange reaction of sulfonyl diamide"**

## Beschreibung

Es ist bekannt, daß heterocyclisch substituierte N-Sulfonamidosulfonylharnstoffe herbizide und pflanzenwachstumsregulierende Eigenschaften aufweisen (EP-A-131 258 oder US-A-4601747).

Die Wirkungen dieser Verbindungen sind jedoch nicht immer befriedigend.

Es wurden nun neue heterocyclische N-Sulfonyldiamidosulfonamide gefunden, die als Herbizide und Pflanzenwachstumsregulatoren besonders geeignet sind.

Gegenstand der vorliegenden Erfindung sind daher Verbindungen der Formel (I) oder deren Salze

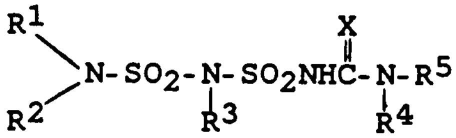

(I)

worin

$R^1$ und $R^2$ unabhängig voneinander H, $(C_1\text{-}C_8)$Alkyl, $(C_2\text{-}C_8)$Alkenyl, $(C_2\text{-}C_8)$Alkinyl, wobei diese Reste gegebenenfalls ein- oder mehrfach durch Halogen oder ein- oder zweifach durch $(C_1\text{-}C_4)$Alkoxy oder $(C_1\text{-}C_4)$Alkylthio substituiert sein können, $(C_1\text{-}C_4)$Alkoxycarbonyl$(C_1\text{-}C_3)$-alkyl, Phenyl, das unsubstituiert oder ein- oder mehrfach durch $(C_1\text{-}C_4)$Alkyl, $(C_1\text{-}C_4)$-Alkoxy, $(C_1\text{-}C_4)$Alkylthio, $(C_1\text{-}C_4)$Alkylsulfinyl, $(C_1\text{-}C_4)$Alkylsulfonyl, wobei die vorgenannten Reste im Alkylteil mehrfach durch Halogen oder ein- oder zweifach durch $(C_1\text{-}C_4)$Alkoxy substituiert sein können, $(C_1\text{-}C_4\text{-Alkoxy})$carbonyl, Halogen oder $NO_2$, substituiert ist, oder $R^1$ und $R^2$ zusammen mit dem sie verknüpfenden N-Atom einen heterocyclischen Ring der Formel

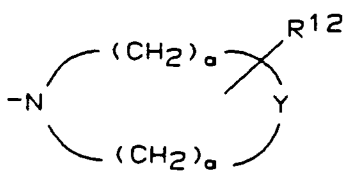

wobei a = unabhängig voneinander eine ganze Zahl von 1 bis 3, bedeutet,

$R^3$ H, $(C_1\text{-}C_{12})$Alkyl, $(C_2\text{-}C_8)$Alkenyl, $(C_2\text{-}C_8)$Alkinyl, wobei diese Reste ein- oder mehrfach durch Halogen oder ein- oder zweifach durch $(C_1\text{-}C_4)$Alkoxy oder $(C_1\text{-}C_4)$Alkylthio substituiert sein können, $(C_1\text{-}C_4\text{-Alkoxy})$carbonyl$(C_1\text{-}C_3)$alkyl, $-(CH_2)_b$Phenyl, wobei b = eine ganze Zahl von 0 bis 2 bedeutet und der Phenylrest unsubstituiert oder ein- oder mehrfach durch $(C_1\text{-}C_4)$Alkyl, $(C_1\text{-}C_4)$Alkoxy, $(C_1\text{-}C_4)$Alkylthio, $(C_1\text{-}C_4)$Alkylsulfonyl, wobei die vorgenannten Reste im Alkylteil ein- oder mehrfach durch Halogen substituiert sein können, durch $(C_1\text{-}C_4)$Alkoxycarbonyl, Halogen oder $NO_2$ substituiert ist, oder $R^2$ und $R^3$ zusammen eine $(C_2\text{-}C_4)$Alkylenkette,

$R^4$ H, $(C_1\text{-}C_6)$Alkyl, $(C_2\text{-}C_8)$Alkenyl, $(C_2\text{-}C_8)$Alkinyl oder $(C_1\text{-}C_4)$Alkoxy,

$R^5$ einen Rest der Formel

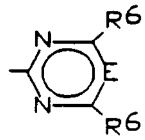 , 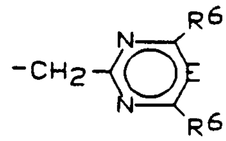 , 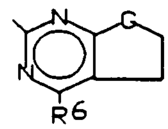 ,

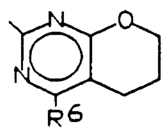 , 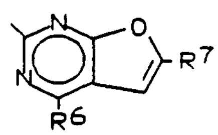 , 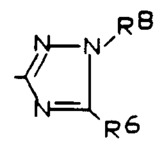 ,

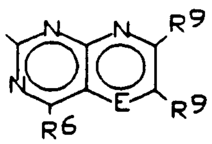 oder 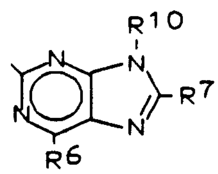

E CH oder N,

G $CH_2$ oder O,

$R^6$ unabhängig voneinander H, Halogen, $(C_1-C_6)$Alkyl, $(C_1-C_6)$Alkoxy oder $(C_1-C_6)$Alkylthio, wobei die vorgenannten Reste im Alkylteil ein- oder mehrfach durch Halogen oder ein- oder zweifach durch $(C_1-C_4)$Alkoxy oder $(C_1-C_4)$Alkylthio substituiert sein können; einen Rest $N(R^{11})_2$, $(C_3-C_6)$Cycloalkyl, $-OCHR^7COOR^{11}$, $(C_2-C_5)$Alkenyl, $(C_2-C_4)$Alkinyl, $(C_3-C_5)$-Alkenyloxy oder $(C_3-C_5)$Alkinyloxy,

$R^7$ H oder $(C_1-C_4)$Alkyl,

$R^8$ $(C_1-C_4)$Alkyl, $-CHF_2$ oder $-CH_2CF_3$,

$R^9$ unabhängig voneinander H, $(C_1-C_4)$Alkyl, $(C_1-C_4)$Alkoxy oder Halogen,

$R^{10}$ H, $(C_1-C_4)$Alkyl, $-CHF_2$ oder $-CH_2CF_3$,

$R^{11}$ unabhängig voneinander H, $(C_1-C_4)$Alkyl, $(C_2-C_4)$Alkenyl oder $(C_3-C_4)$Alkinyl,

$R^{12}$ H, $(C_1-C_4)$Alkyl, $(C_1-C_4$-Alkoxy)carbonyl, $(C_1-C_4)$Alkoxymethyl,

X O oder S und

Y O, S, $CH_2$, NH oder $N(C_1-C_4)$Alkyl bedeuten.

Die Verbindungen der Formel I können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali-, oder Erdalkali-, (gegebenenfalls alkylierte) Ammonium- oder organische Aminsalze. Sie werden vorzugsweise in inerten Lösungsmitteln wie z.B. Wasser, Methanol oder Aceton bei Temperaturen von 0-100 °C aus den Verbindungen der Formel I hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Bevorzugte Verbindungen der Formel I sind solche, bei denen $R^1$ und $R^2$ = unabhängig voneinander H, $(C_1-C_4)$Alkyl oder $R^1$ und $R^2$ zusammen einen heterocyclischen Ring der Formel

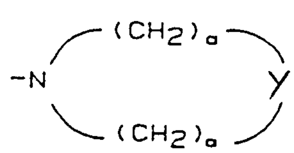

wobei

$R^3$ H, $(C_1-C_6)$Alkyl oder $(C_2-C_4)$Alkenyl,

$R^4$ H, $(C_1-C_4)$Alkyl oder Allyl,

$R^5$ einen Rest der Formel

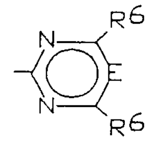

| | |
|---|---|
| $R^6$ | unabhängig voneinander Halogen, $(C_1\text{-}C_4)$Alkyl oder $(C_1\text{-}C_4)$Alkoxy, die beide halogeniert sein können, |
| E | CH oder N, |
| Y | O und |
| a | eine ganze Zahl von 1 bis 3 bedeuten. |

Halogen bedeutet insbesondere F, Cl oder Br. Halogeniertes $(C_1\text{-}C_4)$Alkyl oder halogeniertes $(C_1\text{-}C_4)$-Alkoxy bedeuten insbesondere $CF_3$, $-CH_2CH_2Cl$, $-CH_2CH_2Br$, $-CH_2CH_2CH_2Cl$, $-CF_2CF_2H$, $-CF_2CFClH$, $-CH_2CHFCF_3$, $-OCHF_2$, $OCF_3$, $OCH_2CF_3$, $-OCH_2CH_2Cl$.

Ein weiterer Gegenstand der vorliegenden Erfindung sind Verfahren zur Herstellung der Verbindungen der allgemeinen Formel (I) oder deren Salze, dadurch gekennzeichnet, daß man

(a) eine Verbindung der Formel (II)

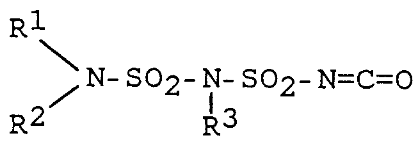 (II),

wobei $R^1$, $R^2$, $R^3$ die für Formel I angegebene Bedeutung außer H besitzen, mit einer Verbindung der Formel (III)

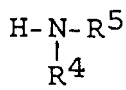 (III)

umsetzt, oder

(b) eine Verbindung der Formel (IV)

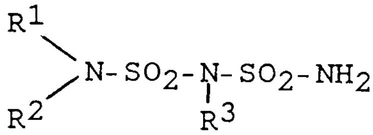 (IV)

wobei $R^1$, $R^2$, $R^3$ die für Formel I angegebene Bedeutung besitzen, mit einem (Thio)-Carbamat der Formel (V)

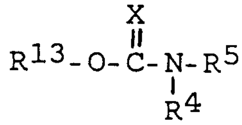 (V),

wobei $R^{13}$ = $(C_1\text{-}C_6)$Alkyl, $(C_1\text{-}C_4)$Halogenalkyl oder Phenyl, das unsubstituiert oder ein- oder mehrfach durch Halogen, $(C_1\text{-}C_4)$Alkyl oder $NO_2$ substituiert ist, umsetzt, oder

(c) eine Verbindung der Formel (VI)

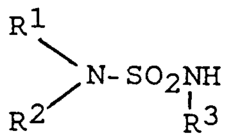 (VI)

wobei $R^1$, $R^2$, $R^3$ die für Formel I angegebene Bedeutung besitzen, mit einem Chlorsulfonylharnstoff der Formel (VII)

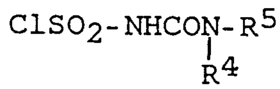 (VII)

wobei $R^4$ und $R^5$ die für Formel I angegebene Bedeutung besitzen, umsetzt, und die unter a), b) oder c) erhaltenen Verbindungen gegebenenfalls in ihre Salze überführt.

**Verfahrensvariante a)**

Die Umsetzung der Verbindungen (II) und (III) erfolgt vorzugsweise in inerten aprotischen Lösungsmitteln, wie z.B. Acetonitril, Dichlormethan, Toluol, Tetrahydrofuran oder Dioxan bei Temperaturen zwischen 0 °C und der Siedetemperatur des Lösungsmittels.

Die Sulfondiamidosulfonyliso(thio)cyanate der Formel (II) sind neu und lassen für X = O sich durch Umsetzung von Chlorsulfonylisocyanat (VIII) mit Sulfonyldiamiden der Formel (VI) wobei $R^1$, $R^2$, $R^3$ die oben angegebene Bedeutung außer Wasserstoff haben in Analogie zu der literaturbekannten Umsetzung von Chlorsulfonylisocyanat mit sekundären Sulfonamiden herstellen (DE-A 2,257,240 oder US-A-3,931,277).

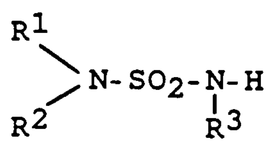

(VI)

$ClSO_2NCO$ (VIII)

Die Sulfonyldiamide der Formel (VI) sind bekannt oder lassen sich nach im Prinzip bekannten Verfahren herstellen, s. z.B. Arch. Pharm. 314, 51 (1980), Ann. Chem. 729, 40 (1969), J. Amer. Chem. Soc. 66, 1242 (1944), Chem. Ber. 111, 1915 (1978).

Die Ausgangsstoffe der Formel (III) sind bekannt oder lassen sich nach im Prinzip bekannten Verfahren herstellen, z.B. durch Cyclisierung entsprechender Guanidinderivate mit entsprechend substituierten 1,3-Diketonen, s. z.B. "The Chemistry of Heterocyclic Compounds", Vol. XVI (1962) and Supplement I (1970), oder durch Derivatisierung von Cyanurchlorid, s. z.B. "The Chemistry of Heterocyclic Compounds", L. Rapaport: "s-Triazines and Derivatives" (1959).

**Verfahrensvariante b)**

Die Umsetzung der Verbindung (IV) mit den heterocyclischen Carbamaten der Formel (V) wird vorzugsweise in Gegenwart von tertiären organischen Basen wie z.B. 1,8-Diazabicyclo-5,4,0-undec-7-en(DBU) in inerten Lösungsmitteln wie Acetonitril oder Dioxan bei Temperaturen zwischen 20 °C und der Siedetemperatur des Lösungsmittels durchgeführt (analog EP-A 44 807).

Die hierfür benötigen Sulfonamide der Formel (IV) sind neu und lassen sich durch Reaktion der Sulfondiamide der Formel (VI), wobei $R^1$, $R^2$ und $R^3$ die für Formel I genannte Bedeutung besitzen, mit Amidosulfochlorid ($ClSO_2NH_2$) herstellen.

Die Reaktion wird in Analogie zu der bekannten Umsetzung von Amidosulfochlorid mit Phenolen durchgeführt, wobei anstelle des Phenols das Sulfondiamid (VI) unter sonst. gleichen Reaktionsbedingungen eingesetzt werden kann s. J. Chem. Soc. Perkin I, 678 (1982), Phosphorus and Sulfur Vol. 20, 371 (1984).

Die Carbamate der Formel (V) sind literaturbekannt oder werden nach bekannten Verfahren hergestellt (EP-A 70 804).

**Verfahrensvariante c)**

Die Umsetzung der Verbindungen (VI) und (VII) erfolgt vorzugsweise in inerten aprotischen Lösungsmitteln, wie z.B. Acetonitril, Dichlormethan, Toluol, Tetrahydrofuran oder Dioxan bei Temperaturen zwischen -78 °C und der Siedetemperatur des Lösungsmittels.

Die Chlorsulfonylharnstoffe der Formel (VII) sind bekannt oder lassen sich nach im Prinzip bekannten Verfahren herstellen (s. z.B. EP-A 39 239).

Die erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt. Bei dikotylen Unkrautarten erstreckt sich das Wirkungsspektrum auf Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den perennierenden Unkräutern.

Unter den spezifischen Kulturbedingungen im Reis vorkommende Unkräuter wie z.B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoff auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Das weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, Emulsionen, versprühbare Lösungen, Stäubemittel, Beizmittel, Dispersionen, Granulate oder Mikrogranulate in den üblichen Zubereitungen angewendet werden. Der Wirkstoffgehalt kann in einem weiten Bereich variieren und beträgt in der Regel 2 bis 95 Gew.%, wobei optimale Gehalte von der Art der Formulierung abhängen.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:
Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Diese oben genannten Formulierungstypen werden beispielsweise beschrieben in:
Winnacker-Küchler, "Chemische Technologie", Band 7, C. Hauser Verlag München, 4. Aufl. 1986; van Falkenberg, "Pesticide Formulations", Marcel Dekker N.Y., 2nd Ed. 1972-73; K. Martens, "Spray Drying Handbook", 3rd Ed. 1979, G. Goodwin Ltd. London.

Die für diese Formulierungen zu verwendenden Formulierungshilfsmittel (Inertmaterialien, Emulgatoren, Netzmittel, Tenside, Lösungsmittel etc.) sind beispielsweise in Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood oder "Encyclopedia of Surface Active Agents", Chem. Publ. Co. Inc., N.Y. 1964 beschrieben.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer

Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

**Beispiel 1**

N-[(4,6-Dimethyl-pyrimidin-2-yl)aminocarbonyl]-N'-(dimethylaminosulfonyl)methylaminosulfonamid

a) N,N,N'-Trimethylsulfonyldiamido-N'-sulfonylisocyanat

28 g (0,20 Mol) Chlorsulfonylisocyanat werden in 300 ml abs. Chlorbenzol gelöst und bei 0 °C mit 25,6 g (0,185 Mol) N,N,N'-Trimethylsulfonyldiamid (hergestellt nach Arch. Pharm. 314, 51 (1980)) - gelöst in 100 ml Chlorbenzol tropfenweise versetzt. Man rührt 1 Std. bei 0 °C und erhitzt das Reaktionsgemisch unter Durchleiten von Stickstoff ca. 2 Std. auf Rückflußtemperatur. Nach dem Abkühlen auf Raumtemperatur wird von Ungelöstem dekantiert und das Reaktionsgemisch unter Destillation im Vakuum eingeengt. Nach Dünnschichtdestillation bei 105 °C/0,2 mbar erhält man ein farbloses Öl, welches ohne weitere Reinigung in der nächsten Stufe eingesetzt wird.

b) N-[(4,6-Dimethoxy-pyrimidin-2-yl)aminocarbonyl]-N'-(dimethylaminosulfonyl)methylaminosulfonamid

3,26 g (0,021 Mol) 2-Amino-4,6-dimethoxy-pyrimidin werden in 80 ml abs. Dichlormethan gelöst und bei 0 °C mit 5,10 g (0,021 Mol) N,N,N'-Trimethylsulfonyldiamido-N'-sulfonylisocyanat - gelöst in 20 ml Dichlormethan versetzt. Man rührt 1 Std. bei 0 °C, 18 h bei Raumtemperatur und 2 Std. bei 40 °C. Nach dem Abkühlen wird das Reaktionsgemisch zweimal mit 0,5 N Salzsäure und anschließend mit gesättigter Kochsalzlösung gewaschen. Nach dem Ausfällen mit n-Heptan wird das Rohprodukt aus $CH_2Cl_2$/n-Heptan umkristallisiert. Man erhält 6,2 g (74,2 % d. Th.) an N-[(4,6-Dimethoxy-pyrimidin-2-yl)-aminocarbonyl]-N'-(dimethylaminosulfonyl)methylaminosulfonamid von Schmp. 140-142 °C.

**Beispiel 2**

N-[(4,6-Dimethoxypyrimidin-2-yl)aminocarbonyl]-N'-(dimethylaminosulfonyl)methylaminosulfonamid

2,41 g (0,017 Mol) Chlorsulfonylisocyanat werden in 80 ml abs. Acetonitril gelöst und bei -40 °C mit 2,1 g (0,017 Mol) 2-Amino-4,6-dimethylpyrimidin versetzt. Man läßt das Reaktionsgemisch in ca. 1 Std. auf 0 °C erwärmen und tropft anschließend bei -40 °C 2,35 g (0,017 Mol) N,N,N'-Trimethylsulfondiamid hinzu. Nach Zugabe von 2,4 ml (0,017 Mol) Triethylamin - gelöst in 20 ml Acetonitril - läßt man die Suspension in ca. 4 Std. auf Raumtemperatur erwärmen. Nach 18 Std. Rühren bei 20 °C wird das Gemisch im Vakuum eingeengt, in Dichlormethan aufgenommen, je zweimal mit 0,5 N Salzsäure und gesättigter Kochsalzlösung gewaschen, über $Na_2SO_4$ getrocknet und die Dichlormethanlösung eingeengt. Durch Zugabe von n-Heptan wird das Rohprodukt ausgefällt und aus Dichlormethan/n-Heptan umkristallisiert. Man erhält 4,76 g (76,5 % d. Th.) an N-[(4,6-Dimethylpyrimidin-2-yl)aminocarbonyl]-N'(dimethylaminosulfonyl)methylaminosulfonamid vom Schmp. 137-139 °C.

Analog zu den in obigen Beispielen beschriebenen Verfahrensweisen lassen sich die Verbindungen der nachfolgenden Tabelle 1 herstellen.

**Tabelle 1**

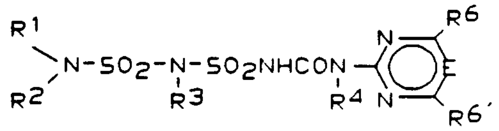

| Bsp. | $R^1$ | $R^2$ | $R^3$ | $R^4$ | E | $R^6$ | $R^{6'}$ | Fp. [°C] |
|---|---|---|---|---|---|---|---|---|
| 3 | H | $CH_3$ | $CH_3$ | H | CH | $CH_3$ | $CH_3$ | |
| 4 | H | $CH_3$ | $CH_3$ | H | CH | $OCH_3$ | $CH_3$ | |
| 5 | H | $CH_3$ | $CH_3$ | H | CH | $OCH_3$ | $OCH_3$ | |
| 6 | H | $CH_3$ | $CH_3$ | H | CH | $OCH_3$ | Cl | |
| 7 | H | $CH_3$ | $CH_3$ | $CH_3$ | CH | $OCH_3$ | $OCH_3$ | 84-88 |
| 8 | H | $CH_3$ | $CH_3$ | H | N | $OCH_3$ | $CH_3$ | |
| 9 | H | $CH_3$ | $CH_3$ | $CH_3$ | N | $OCH_3$ | $CH_3$ | |
| 10 | H | $CH_3$ | $CH_3$ | H | N | $OCH_3$ | $OCH_3$ | |
| 11 | H | $CH_3$ | $CH_3$ | H | N | $OCH_3$ | $NHCH_3$ | |
| 12 | H | $CH_3$ | $CH_3$ | H | CH | $OCH_3$ | $OCH_2CF_3$ | |
| 13 | H | $CH_3$ | $CH_3$ | H | N | $OCH_3$ | $OCH_2CF_3$ | |
| 14 | H | $CH_3$ | $CH_3$ | H | CH | $OCHF_2$ | $CH_3$ | |
| 15 | H | $CH_3$ | $CH_3$ | H | CH | $OCHF_2$ | $OCHF_2$ | |
| 16 | H | $CH_3$ | $CH_3$ | H | N | $OC_2H_5$ | $NHCH_3$ | |
| 17 | H | $CH_3$ | $CH_3$ | H | N | $OCH_3$ | $N(CH_3)_2$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 18 | H | H | $CH_3$ | H | CH | $OCH_3$ | $OCH_3$ | |
| 19 | H | H | H | H | CH | $OCH_3$ | $OCH_3$ | |
| 20 | $CH_3$ | $CH_3$ | H | H | CH | $OCH_3$ | $OCH_3$ | 182-183°C |
| 21 | $CH_3$ | $CH_3$ | $CH_3$ | H | CH | $OCH_3$ | $CH_3$ | 162-164°C |
| 22 | $CH_3$ | $CH_3$ | $CH_3$ | $CH_3$ | CH | $OCH_3$ | $OCH_3$ | 102-104°C |
| 23 | $CH_3$ | $CH_3$ | $CH_3$ | H | CH | Cl | $OCH_3$ | 142°C |
| 24 | $CH_3$ | $CH_3$ | $CH_3$ | H | CH | $OCHF_2$ | $OCHF_2$ | |
| 25 | $CH_3$ | $CH_3$ | $CH_3$ | H | CH | Cl | $CH_3$ | |
| 26 | $CH_3$ | $CH_3$ | $CH_3$ | H | CH | $OCH_3$ | $NHCH_3$ | |
| 27 | $CH_3$ | $CH_3$ | $CH_3$ | H | CH | $OCH_3$ | $N(CH_3)_2$ | |
| 28 | $CH_3$ | $CH_3$ | $CH_3$ | H | CH | $OC_2H_5$ | $OCH_3$ | |
| 29 | $CH_3$ | $CH_3$ | $CH_3$ | H | CH | $OCH_3$ | $SCH_3$ | |
| 30 | $CH_3$ | $CH_3$ | $CH_3$ | H | N | $CH_3$ | $CH_3$ | |
| 31 | $CH_3$ | $CH_3$ | $CH_3$ | H | N | $OCH_3$ | $CH_3$ | |
| 32 | $CH_3$ | $CH_3$ | $CH_3$ | H | N | $OCH_3$ | $OCH_3$ | |
| 33 | $CH_3$ | $CH_3$ | $CH_3$ | $CH_3$ | N | $OCH_3$ | $CH_3$ | 69-70°C |
| 34 | $CH_3$ | $CH_3$ | $CH_3$ | $CH_3$ | N | $OCH_3$ | $OCH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 35 | $CH_3$ | $CH_3$ | $CH_3$ | H | N | $OCH_3$ | 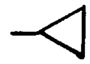 | |
| 36 | $CH_3$ | $CH_3$ | $CH_3$ | H | N | $OCH_3$ | $NHCH_3$ | |
| 37 | $CH_3$ | $CH_3$ | $CH_3$ | H | N | $OC_2H_5$ | $NHCH_3$ | |
| 38 | $CH_3$ | $CH_3$ | $CH_3$ | H | N | $OCH_3$ | $NHC_2H_5$ | |
| 39 | $CH_3$ | $CH_3$ | $CH_3$ | H | N | $CH_3$ | $SCH_3$ | |
| 40 | $CH_3$ | $CH_3$ | $CH_3$ | H | N | $OCH_3$ | $N(CH_3)_2$ | |
| 41 | $CH_3$ | $CH_3$ | $C_2H_5$ | H | CH | $CH_3$ | H | |
| 42 | $CH_3$ | $CH_3$ | $C_2H_5$ | H | CH | $CH_3$ | $CH_3$ | |
| 43 | $CH_3$ | $CH_3$ | $C_2H_5$ | H | CH | $OCH_3$ | $CH_3$ | |
| 44 | $CH_3$ | $CH_3$ | $C_2H_5$ | H | CH | Cl | $CH_3$ | |
| 45 | $CH_3$ | $CH_3$ | $C_2H_5$ | H | CH | $OCH_3$ | $OCH_3$ | 148-150°C |
| 46 | $CH_3$ | $CH_3$ | $C_2H_5$ | H | CH | Cl | $OCH_3$ | |
| 47 | $CH_3$ | $CH_3$ | $C_2H_5$ | H | CH | $OCF_2H$ | $OCF_2H$ | |
| 48 | $CH_3$ | $CH_3$ | $C_2H_5$ | $CH_3$ | CH | $OCH_3$ | $OCH_3$ | 130-137°C |
| 49 | $CH_3$ | $CH_3$ | $C_2H_5$ | H | CH | $OCF_2H$ | $CH_3$ | |
| 50 | $CH_3$ | $CH_3$ | $C_2H_5$ | H | CH | $OCH_3$ | $NHCH_3$ | |
| 51 | $CH_3$ | $CH_3$ | $C_2H_5$ | H | CH | $OCH_3$ | $N(CH_3)_2$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 52 | $CH_3$ | $CH_3$ | $C_2H_5$ | H | N | $CH_3$ | $CH_3$ | |
| 53 | $CH_3$ | $CH_3$ | $C_2H_5$ | H | CH | $OCH_3$ | 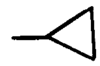 | |
| 54 | $CH_3$ | $CH_3$ | $C_2H_5$ | H | N | $OCH_3$ | $CH_3$ | |
| 55 | $CH_3$ | $CH_3$ | $C_2H_5$ | $CH_3$ | N | $OCH_3$ | $CH_3$ | |
| 56 | $CH_3$ | $CH_3$ | $C_2H_5$ | $CH_3$ | N | $OCH_3$ | $OCH_3$ | |
| 57 | $CH_3$ | $CH_3$ | $C_2H_5$ | H | N | $OCH_3$ | $OCH_3$ | |
| 58 | $CH_3$ | $CH_3$ | $C_2H_5$ | H | N | $OCH_3$ | $OCH_2CF_3$ | |
| 59 | $CH_3$ | $CH_3$ | $C_2H_5$ | H | N | $CH_3$ | $OCH_2CF_3$ | |
| 60 | $CH_3$ | $CH_3$ | $C_2H_5$ | H | N | $OCH_3$ | 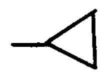 | |
| 61 | $CH_3$ | $CH_3$ | $C_3H_7(n)$ | H | CH | $CH_3$ | $CH_3$ | |
| 62 | $CH_3$ | $CH_3$ | $C_3H_7(n)$ | H | CH | $OCH_3$ | $CH_3$ | |
| 63 | $CH_3$ | $CH_3$ | $C_3H_7(n)$ | H | CH | $OCH_3$ | $OCH_3$ | |
| 64 | $CH_3$ | $CH_3$ | $C_3H_7(n)$ | H | CH | Cl | $OCH_3$ | |
| 65 | $CH_3$ | $CH_3$ | $C_3H_7(n)$ | H | N | $OCH_3$ | $CH_3$ | |
| 66 | $CH_3$ | $CH_3$ | $C_3H_7(n)$ | $CH_3$ | CH | $OCH_3$ | $OCH_3$ | 136-138°C |
| 67 | $CH_3$ | $CH_3$ | $C_3H_7(n)$ | $CH_3$ | CH | $OCH_3$ | $CH_3$ | |
| 68 | $CH_3$ | $CH_3$ | $C_3H_7(n)$ | H | N | $OCH_3$ | $OCH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 69 | $CH_3$ | $CH_3$ | 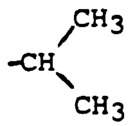 | H | CH | $CH_3$ | $CH_3$ | |
| 70 | $CH_3$ | $CH_3$ | " | H | CH | $OCH_3$ | $CH_3$ | |
| 71 | $CH_3$ | $CH_3$ | " | H | CH | $OCH_3$ | $OCH_3$ | |
| 72 | $CH_3$ | $CH_3$ | " | $CH_3$ | CH | $OCH_3$ | $OCH_3$ | |
| 73 | $CH_3$ | $CH_3$ | " | H | N | $OCH_3$ | $CH_3$ | |
| 74 | $CH_3$ | $CH_3$ | " | H | N | $OCH_3$ | $OCH_3$ | |
| 75 | $CH_3$ | $CH_3$ | $-C_4H_9(n)$ | H | CH | $CH_3$ | $CH_3$ | |
| 76 | $CH_3$ | $CH_3$ | " | H | CH | $OCH_3$ | $CH_3$ | |
| 77 | $CH_3$ | $CH_3$ | " | H | CH | Cl | $OCH_3$ | |
| 78 | $CH_3$ | $CH_3$ | " | H | CH | $OCH_3$ | $OCH_3$ | |
| 79 | $CH_3$ | $CH_3$ | " | $CH_3$ | CH | $OCH_3$ | $OCH_3$ | |
| 80 | $CH_3$ | $CH_3$ | " | H | N | $OCH_3$ | $CH_3$ | |
| 81 | $CH_3$ | $CH_3$ | " | H | N | $OCH_3$ | $OCH_3$ | |
| 82 | $CH_3$ | $CH_3$ | 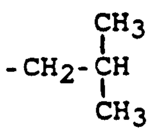 | H | CH | $CH_3$ | $CH_3$ | |
| 83 | $CH_3$ | $CH_3$ | " | H | CH | $OCH_3$ | $OCH_3$ | |
| 84 | $CH_3$ | $CH_3$ | " | H | N | $OCH_3$ | $CH_3$ | |
| 85 | $CH_3$ | $CH_3$ | " | $CH_3$ | N | $OCH_3$ | $OCH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 86 | $CH_3$ | $CH_3$ | $-(CH_2)_5CH_3$ | H | CH | $OCH_3$ | $OCH_3$ | |
| 87 | $CH_3$ | $CH_3$ | $-C(CH_3)_3$ | H | CH | $OCH_3$ | $OCH_3$ | |
| 88 | $CH_3$ | $CH_3$ | $-(CH_2)_9CH_3$ | H | CH | $OCH_3$ | $OCH_3$ | |
| 89 | $CH_3$ | $CH_3$ | $-(CH_2)_{11}CH_3$ | H | CH | $OCH_3$ | Cl | |
| 90 | $CH_3$ | $CH_3$ | $-CH_2CH=CH_2$ | H | CH | $OCH_3$ | $OCH_3$ | |
| 91 | $CH_3$ | $CH_3$ | $-CH_2C\equiv CH$ | H | CH | $OCH_3$ | $OCH_3$ | |
| 92 | $CH_3$ | $CH_3$ | $-CH_2CH=\underset{\mid\atop CH_3}{CH}$ | H | CH | $OCH_3$ | $OCH_3$ | |
| 93 | $CH_3$ | $CH_3$ | $-CH_2CH_2Cl$ | H | CH | $OCH_3$ | $OCH_3$ | |
| 94 | $CH_3$ | $CH_3$ | $-CH_2CH_2OCH_3$ | H | CH | $OCH_3$ | $OCH_3$ | |
| 95 | $CH_3$ | $CH_3$ | $-CH_2CH_2SCH_3$ | H | CH | $OCH_3$ | $OCH_3$ | |
| 96 | $CH_3$ | $CH_3$ | $-CH_2CH_2SC_2H_5$ | H | CH | $OCH_3$ | $OCH_3$ | |
| 97 | $CH_3$ | $CH_3$ | $-CH_2COOCH_3$ | H | CH | $OCH_3$ | $OCH_3$ | |
| 98 | $CH_3$ | $CH_3$ | $-CH_2COOC_2H_5$ | H | CH | $OCH_3$ | $OCH_3$ | |
| 99 | $CH_3$ | $CH_3$ | $-\underset{\mid\atop CH_3}{C}HCOOCH_3$ | H | CH | $OCH_3$ | $OCH_3$ | |
| 100 | $CH_3$ | $CH_3$ | $-CH_2CH_2COOCH_3$ | H | CH | $OCH_3$ | $OCH_3$ | |
| 101 | $CH_3$ | $CH_3$ | $-C_6H_5$ | H | CH | $OCH_3$ | $CH_3$ | |
| 102 | $CH_3$ | $CH_3$ | $-C_6H_5$ | H | CH | $OCH_3$ | $OCH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 103 | $CH_3$ | $CH_3$ | $-C_6H_4-2-Cl$ | H | CH | $OCH_3$ | $OCH_3$ | |
| 104 | $CH_3$ | $CH_3$ | $-C_6H_4-2-CF_3$ | H | CH | $OCH_3$ | $OCH_3$ | |
| 105 | $CH_3$ | $CH_3$ | $-C_6H_4-2-SO_2CH_3$ | H | CH | $OCH_3$ | $OCH_3$ | |
| 106 | $CH_3$ | $CH_3$ | $-C_6H_4-2-COOCH_3$ | H | CH | $OCH_3$ | $OCH_3$ | |
| 107 | $CH_3$ | $CH_3$ | $-C_6H_4-4-Cl$ | H | CH | $OCH_3$ | $OCH_3$ | |
| 108 | $CH_3$ | $CH_3$ | Benzyl | H | CH | $OCH_3$ | $OCH_3$ | |
| 109 | $CH_3$ | $-(CH_2)_2-$ | | H | CH | $CH_3$ | $CH_3$ | |
| 110 | $CH_3$ | $-(CH_2)_2-$ | | H | CH | $CH_3$ | $OCH_3$ | |
| 111 | $CH_3$ | $-(CH_2)_2-$ | | H | CH | $OCH_3$ | $OCH_3$ | 140-142 |
| 112 | $CH_3$ | $-(CH_2)_2-$ | | H | CH | $OCH_3$ | Cl | |
| 113 | $CH_3$ | $-(CH_2)_2-$ | | H | N | $OCH_3$ | $CH_3$ | |
| 114 | $CH_3$ | $-(CH_2)_2-$ | | $CH_3$ | N | $OCH_3$ | $CH_3$ | |
| 115 | $CH_3$ | $-(CH_2)_2-$ | | H | N | $OCH_3$ | $OCH_3$ | |
| 116 | $CH_3$ | $-(CH_2)_2-$ | | $CH_3$ | N | $OCH_3$ | $OCH_3$ | |
| 117 | $CH_3$ | $-(CH_2)_2-$ | | H | CH | $CH_3$ | $CH_3$ | |
| 118 | $CH_3$ | $-(CH_2)_2-$ | | H | CH | $OCH_3$ | $OCH_3$ | |
| 119 | $CH_3$ | $-(CH_2)_2-$ | | H | N | $OCH_3$ | $CH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 120a | $CH_3$ | $-(CH_2)_4-$ | | H | CH | $CH_3$ | $CH_3$ | |
| 120b | $CH_3$ | $-(CH_2)_3-$ | | H | CH | $CH_3$ | $CH_3$ | |
| 121a | $CH_3$ | $-(CH_2)_4-$ | | H | CH | $OCH_3$ | $OCH_3$ | |
| 121b | $CH_3$ | $-(CH_2)_3-$ | | H | CH | $OCH_3$ | $OCH_3$ | |
| 122a | $CH_3$ | $-(CH_2)_4-$ | | H | N | $OCH_3$ | $CH_3$ | |
| 122b | $CH_3$ | $-(CH_2)_3-$ | | H | N | $OCH_3$ | $CH_3$ | |
| 123 | $C_2H_5$ | H | $CH_3$ | H | CH | $OCH_3$ | $OCH_3$ | |
| 124 | $C_2H_5$ | H | $C_2H_5$ | H | CH | $OCH_3$ | $OCH_3$ | |
| 125 | $C_2H_5$ | $CH_3$ | $CH_3$ | H | CH | $OCH_3$ | $OCH_3$ | |
| 126 | $C_2H_5$ | $CH_3$ | H | H | CH | $OCH_3$ | $OCH_3$ | |
| 127 | $C_2H_5$ | $C_2H_5$ | H | H | CH | $OCH_3$ | $OCH_3$ | |
| 128 | $C_2H_5$ | $C_2H_5$ | $CH_3$ | H | CH | $CH_3$ | $CH_3$ | |
| 129 | $C_2H_5$ | $C_2H_5$ | $CH_3$ | H | CH | $OCH_3$ | $CH_3$ | |
| 130 | $C_2H_5$ | $C_2H_5$ | $CH_3$ | H | CH | $OCH_3$ | $OCH_3$ | 116-118°C |
| 131 | $C_2H_5$ | $C_2H_5$ | $CH_3$ | $CH_3$ | CH | $OCH_3$ | $OCH_3$ | |
| 132 | $C_2H_5$ | $C_2H_5$ | $CH_3$ | H | CH | Cl | $OCH_3$ | |
| 133 | $C_2H_5$ | $C_2H_5$ | $CH_3$ | H | N | $CH_3$ | $CH_3$ | |
| 134 | $C_2H_5$ | $C_2H_5$ | $CH_3$ | H | N | $OCH_3$ | $CH_3$ | |
| 135 | $C_2H_5$ | $C_2H_5$ | $CH_3$ | $CH_3$ | N | $OCH_3$ | $CH_3$ | |
| 136 | $C_2H_5$ | $C_2H_5$ | $CH_3$ | H | N | $OCH_3$ | $OCH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 137 | n-$C_3H_7$ | $CH_3$ | $CH_3$ | H | CH | $OCH_3$ | $OCH_3$ | |
| 138 | n-$C_4H_9$ | $CH_3$ | $CH_3$ | H | CH | $OCH_3$ | $OCH_3$ | |
| 139 | n-$C_5H_{11}$ | $CH_3$ | $CH_3$ | H | CH | $OCH_3$ | $OCH_3$ | |
| 140 | n-$C_6H_{13}$ | $CH_3$ | $CH_3$ | H | CH | $OCH_3$ | $OCH_3$ | |
| 141 | n-$C_8H_{17}$ | $CH_3$ | $CH_3$ | H | CH | $OCH_3$ | $OCH_3$ | |
| 142 | $CH_2$=CH- | $CH_3$ | $CH_3$ | H | CH | $OCH_3$ | $OCH_3$ | |
| 143 | $CH_2$=CH$CH_2$- | $CH_3$ | $CH_3$ | H | CH | $OCH_3$ | $OCH_3$ | |
| 144 | CH≡C$CH_2$- | $CH_3$ | $CH_3$ | H | CH | $OCH_3$ | $OCH_3$ | |
| 145 | Cl$CH_2CH_2$- | $CH_3$ | $CH_3$ | H | CH | $OCH_3$ | $OCH_3$ | |
| 146 | $CH_3OCH_2CH_2$ | $CH_3$ | $CH_3$ | H | CH | $OCH_3$ | $OCH_3$ | |
| 147 | $CH_3SCH_2CH_2$- | $CH_3$ | $CH_3$ | H | CH | $OCH_3$ | $OCH_3$ | |
| 148 | $CH_3OOCCH_2$- | $CH_3$ | $CH_3$ | H | CH | $OCH_3$ | $OCH_3$ | |
| 149 | $C_2H_5OOC$CH($CH_3$)- | $CH_3$ | $CH_3$ | H | CH | $OCH_3$ | $OCH_3$ | |
| 150 | -$C_6H_5$ | $CH_3$ | H | H | CH | $CH_3$ | $CH_3$ | |
| 151 | -$C_6H_5$ | $CH_3$ | $CH_3$ | H | CH | $CH_3$ | $CH_3$ | |
| 152 | -$C_6H_5$ | $CH_3$ | $CH_3$ | H | CH | $OCH_3$ | $CH_3$ | |
| 153 | -$C_6H_5$ | $CH_3$ | $CH_3$ | H | CH | $OCH_3$ | $OCH_3$ | 30-35°C |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 154 | $C_6H_5$- | $CH_3$ | $CH_3$ | H | N | $CH_3$ | $OCH_3$ | |
| 155 | $C_6H_5$- | $CH_3$ | $CH_3$ | $CH_3$ | N | $CH_3$ | $OCH_3$ | |
| 156 | $C_6H_5$ | $CH_3$ | $CH_3$ | H | N | $OCH_3$ | $OCH_3$ | |
| 157 | $-C_6H_4$-2-Cl | $CH_3$ | $CH_3$ | H | N | $OCH_3$ | $OCH_3$ | |
| 158 | $-C_6H_4$-2-$COOCH_3$ | $CH_3$ | $CH_3$ | H | CH | $OCH_3$ | $OCH_3$ | |
| 159 | $-(CH_2)_4-$ | | $CH_3$ | H | CH | $CH_3$ | $CH_3$ | |
| 160 | $-(CH_2)_4-$ | | $CH_3$ | H | CH | $OCH_3$ | $OCH_3$ | 77-80°C |
| 161 | $-(CH_2)_4-$ | | $CH_3$ | H | CH | $OCH_3$ | Cl | |
| 162 | $-(CH_2)_4-$ | | $C_2H_5$ | H | CH | $OCH_3$ | $OCH_3$ | |
| 163 | $-(CH_2)_4-$ | | n-$C_3H_7$ | H | CH | $OCH_3$ | $OCH_3$ | |
| 164 | $-(CH_2)_5-$ | | $CH_3$ | H | CH | $OCH_3$ | $OCH_3$ | |
| 165 | $-(CH_2)_5$ | | $CH_3$ | H | N | $OCH_3$ | $CH_3$ | |
| 166 | $-(CH_2)_5-$ | | $CH_3$ | $CH_3$ | N | $OCH_3$ | $OCH_3$ | |
| 167 | Morpholin-4-yl | | $CH_3$ | H | CH | $CH_3$ | $CH_3$ | |
| 168 | " | | $CH_3$ | H | CH | $OCH_3$ | $OCH_3$ | |
| 169 | " | | $CH_3$ | H | CH | $OCH_3$ | $CH_3$ | |
| 170 | Thiomorpholin-4-yl | | $CH_3$ | H | CH | $OCH_3$ | $OCH_3$ | |

**Fortsetzung von Tabelle 1**

| Bsp. | $R^1$ | $R^2$ | $R^3$ | $R^4$ | E | $R^6$ | $R^{6'}$ | Fp. |
|---|---|---|---|---|---|---|---|---|
| 171 | Piperazin-1-yl | | $CH_3$ | H | CH | $OCH_3$ | $OCH_3$ | |
| 172 | " | | $CH_3$ | H | N | $OCH_3$ | $CH_3$ | |
| 173 | 4-Methyl-piperazin-1-yl | | $CH_3$ | H | CH | $OCH_3$ | $OCH_3$ | |
| 174 | " | | $CH_3$ | H | N | $OCH_3$ | $CH_3$ | |
| 175 | 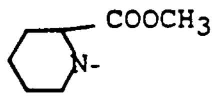 | | $CH_3$ | H | CH | $OCH_3$ | $OCH_3$ | |
| 176 | 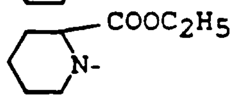 | | $CH_3$ | H | CH | $OCH_3$ | $CH_3$ | |
| 177 | 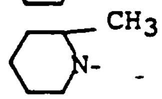 | | $CH_3$ | H | CH | $OCH_3$ | $OCH_3$ | |
| 178 | 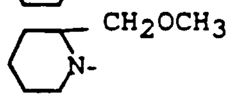 | | $CH_3$ | H | N | $OCH_3$ | $CH_3$ | |
| 179 | 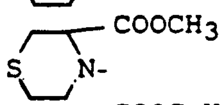 | | $CH_3$ | H | CH | $OCH_3$ | $OCH_3$ | |
| 180 | 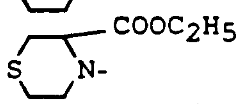 | | $CH_3$ | H | CH | $CH_3$ | $CH_3$ | |
| 181 | 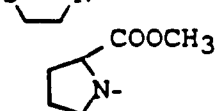 | | $CH_3$ | H | CH | $CH_3$ | $CH_3$ | |
| 182 | 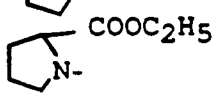 | | $CH_3$ | H | CH | $OCH_3$ | $OCH_3$ | |
| 183 | 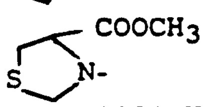 | | $CH_3$ | H | CH | $OCH_3$ | $OCH_3$ | |
| 184 | 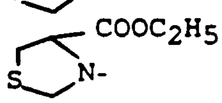 | | $CH_3$ | H | CH | $OCH_3$ | $OCH_3$ | |
| 185 | 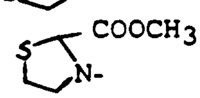 | | $CH_3$ | H | CH | $CH_3$ | $CH_3$ | |
| 186 | 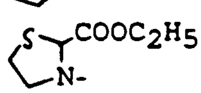 | | $CH_3$ | H | CH | $OCH_3$ | $OCH_3$ | |
| 187 | 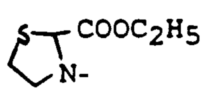 | | $CH_3$ | $CH_3$ | CH | $OCH_3$ | $OCH_3$ | |

**Tabelle 2**

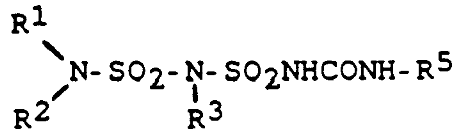

| Bsp. | $R^1$ | $R^2$ | $R^3$ | $R^5$ | Fp |
|---|---|---|---|---|---|
| 188 | $CH_3$ | $CH_3$ | $CH_3$ | 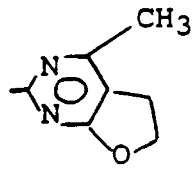 | |
| 189 | $CH_3$ | $CH_3$ | $C_2H_5$ | 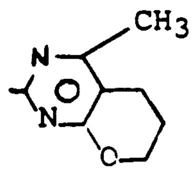 | |
| 190 | $CH_3$ | $CH_3$ | $C_2H_5$ | 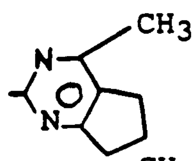 | |
| 191 | $CH_3$ | $CH_3$ | $CH_3$ | 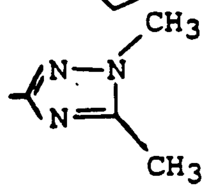 | |
| 192 | $CH_3$ | $CH_3$ | $CH_3$ | 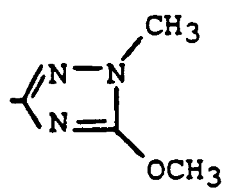 | |
| 193 | $C_2H_5$ | $C_2H_5$ | $CH_3$ | " | |
| 194 | $-(CH_2)_4$ | | $CH_3$ | " | |
| 195 | $-(CH_2)_5$ | | $CH_3$ | " | |
| 196 | $CH_3$ | $-(CH_2)_2-$ | | " | |
| 197 | $CH_3$ | $-(CH_2)_3-$ | | " | |

**Fortsetzung der Tabelle 2**

| Bsp. | $R^1$ | $R^2$ | $R^3$ | $R^5$ | Fp |
|---|---|---|---|---|---|
| 198 | $CH_3$ | $CH_3$ | $CH_3$ | 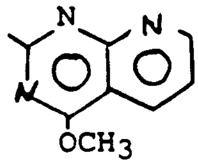 | |
| 199 | $CH_3$ | $CH_3$ | $CH_3$ | 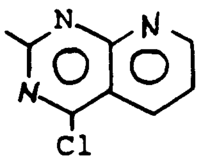 | |
| 200 | $CH_3$ | $CH_3$ | $CH_3$ | 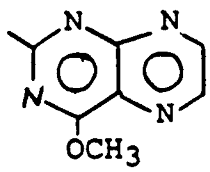 | |
| 201 | $CH_3$ | $CH_3$ | $C_2H_5$ | 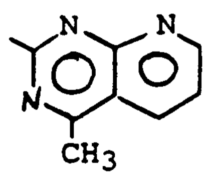 | |
| 202 | $CH_3$ | $CH_3$ | $CH_3$ | " | |
| 203 | $CH_3$ | $CH_3$ | $CH_3$ | 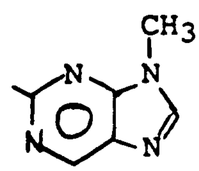 | |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

**1. Unkrautwirkung im Vorauflauf**

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte

nach dem Auflaufen der Versuchspflanzen nach einer Versuchszeit von 3 - 4 Wochen im Vergleich zu unbehandelten Kontrollen. Wie die Boniturwerte in Tabelle 3 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 - 800 l/ha auf die grünen Pflanzenteile gesprüht und nach ca. 3 - 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser une Unkräuter auf (Tabelle 4).

**3. Kulturpflanzenverträglichkeit**

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z. B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

Tabelle 3

| **Vorauflaufwirkung der erfindungsgemäßen Verbindungen** | | | | | |
|---|---|---|---|---|---|
| Bsp. Nr. | Dosierung (kg a.i./ha) | herbizide Wirkung | | | |
| | | SIA | CRS | STM | LOM |
| 1 | 0,6 | 5 | 5 | 5 | 5 |
| 2 | 0,6 | 5 | 5 | 5 | 5 |
| 7 | 0,6 | 5 | 4 | 4 | 5 |
| 20 | 0,6 | 5 | 5 | 5 | 5 |
| 21 | 0,6 | 5 | 5 | 5 | 5 |
| 22 | 0,6 | 5 | 5 | 5 | 5 |
| 23 | 0,6 | 5 | 5 | 5 | 5 |
| 33 | 0,6 | 5 | 5 | 5 | 5 |
| 45 | 0,6 | 5 | 5 | 5 | 5 |
| 48 | 0,6 | 5 | 5 | 5 | 5 |
| 66 | 0,6 | 5 | 5 | 5 | 5 |
| 111 | 0,6 | 5 | 5 | 5 | 5 |
| 130 | 0,6 | 4 | 4 | 4 | 4 |
| 153 | 0,6 | 5 | 5 | 5 | 5 |
| 160 | 0,6 | 5 | 5 | 5 | 5 |

Tabelle 4

| Nachlaufwirkung | | | | | |
|---|---|---|---|---|---|
| Bsp. Nr. | Dosierung (kg a.i./ha) | herbizide Wirkung | | | |
| | | SIA | CRS | STM | LOM |
| 1 | 0,6 | 5 | 5 | 5 | 5 |
| 2 | 0,6 | 5 | 5 | 5 | 5 |
| 7 | 0,6 | 5 | 5 | 4 | 4 |
| 20 | 0,6 | 5 | 5 | 5 | 5 |
| 21 | 0,6 | 5 | 5 | 5 | 5 |
| 22 | 0,6 | 5 | 5 | 5 | 5 |
| 23 | 0,6 | 5 | 5 | 5 | 5 |
| 33 | 0,6 | 5 | 5 | 5 | 5 |
| 45 | 0,6 | 5 | 5 | 5 | 5 |
| 48 | 0,6 | 5 | 5 | 5 | 5 |
| 66 | 0,6 | 5 | 5 | 5 | 5 |
| 111 | 0,6 | 5 | 5 | 5 | 5 |
| 130 | 0,6 | 4 | 4 | 4 | 4 |
| 153 | 0,6 | 5 | 5 | 5 | 5 |
| 160 | 0,6 | 5 | 5 | 5 | 5 |

Abkürzungen:
SIA = Sinapis alba
CRS = Chrysanthemum segetum
STM = Stellaria media
LOM = Lolium multiflorum

**Wuchshemmung an Getreide**

In Schalenversuche im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnass gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100 % den Stillstand des Wachstums und 0 % ein Wachstum entsprechend den unbehandelten Kontrollpflanzen bedeuten. Es zeigte sich, daß die Verbindungen sehr gute wachstumsregulierende Eigenschaften besitzen. Die Ergebnisse sind in der nachfolgenden Tabelle zusammengestellt.

Tabelle 5

| Verbindungen nach Bsp.-Nr. | Anwendungskonz. Kg/ha | Wuchshemmung (%) | | | Phytotox. Wirkung |
|---|---|---|---|---|---|
| | | Weizen | Gerste | Roggen | |
| 1 | 0.62<br>0.31 | 25<br>18 | 24<br>17 | 43<br>29 | keine<br>Schäden |
| 2 | 0.62<br>0.31 | 19<br>15 | 22<br>17 | 35<br>29 | keine<br>Schäden |

**Patentansprüche**

**Patentansprüche für folgende Vertragsstaaten : AT, BE, CH, DE, FR, GB, IT, LI, NL**

1. Verbindungen der Formel I oder deren Salze

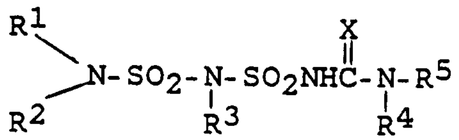

(I)

worin

$R^1$ und $R^2$ unabhängig voneinander H, $(C_1\text{-}C_8)$Alkyl, $(C_2\text{-}C_8)$Alkenyl, $(C_2\text{-}C_8)$Alkinyl, wobei diese Reste gegebenenfalls ein- oder mehrfach durch Halogen oder ein- oder zweifach durch $(C_1\text{-}C_4)$Alkoxy oder $(C_1\text{-}C_4)$Alkylthio substituiert sein können, $(C_1\text{-}C_4)$-Alkoxycarbonyl$(C_1\text{-}C_3)$alkyl, Phenyl, das unsubstituiert oder ein- oder mehrfach durch $(C_1\text{-}C_4)$Alkyl, $(C_1\text{-}C_4)$Alkoxy, $(C_1\text{-}C_4)$Alkylthio, $(C_1\text{-}C_4)$Alkylsulfinyl, $(C_1\text{-}C_4)$-Alkylsulfonyl, wobei die vorgenannten Reste im Alkylteil mehrfach durch Halogen oder ein- oder zweifach durch $(C_1\text{-}C_4)$Alkoxy substituiert sein können, $(C_1\text{-}C_4\text{-Alkoxy})$-carbonyl, Halogen oder $NO_2$, substituiert ist, oder $R^1$ und $R^2$ zusammen mit dem sie verknüpfenden N-Atom einen heterocyclischen Ring der Formel

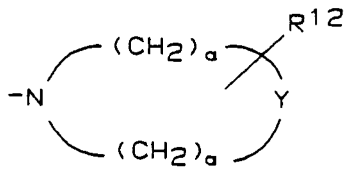

wobei a = unabhängig voneinander eine ganze Zahl von 1 bis 3, bedeutet,

$R^3$ H, $(C_1\text{-}C_{12})$Alkyl, $(C_2\text{-}C_8)$Alkenyl, $(C_2\text{-}C_8)$Alkinyl, wobei diese Reste ein- oder mehrfach durch Halogen oder ein- oder zweifach durch $(C_1\text{-}C_4)$Alkoxy oder $(C_1\text{-}C_4)$Alkylthio substituiert sein können, $(C_1\text{-}C_4\text{-Alkoxy})$carbonyl$(C_1\text{-}C_3)$alkyl, $-(CH_2)_b$Phenyl, wobei b = eine ganze Zahl von 0 bis 2 bedeutet und der Phenylrest unsubstituiert oder ein- oder mehrfach durch $(C_1\text{-}C_4)$Alkyl, $(C_1\text{-}C_4)$Alkoxy, $(C_1\text{-}C_4)$Alkylthio, $(C_1\text{-}C_4)$-Alkylsulfonyl, wobei die vorgenannten Reste im Alkylteil ein- oder mehrfach durch Halogen substituiert sein können, durch $(C_1\text{-}C_4)$Alkoxycarbonyl, Halogen oder $NO_2$ substituiert ist, oder $R^2$ und $R^3$ zusammen eine $(C_2\text{-}C_4)$Alkylenkette,

$R^4$ H, $(C_1\text{-}C_6)$Alkyl, $(C_2\text{-}C_8)$Alkenyl, $(C_2\text{-}C_8)$Alkinyl oder $(C_1\text{-}C_4)$Alkoxy,

$R^5$ einen Rest der Formel

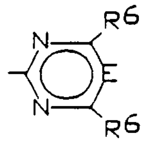 , 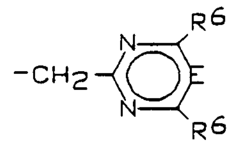 , 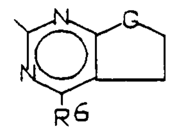 ,

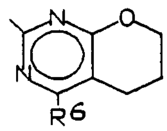 , 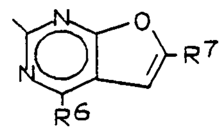 , 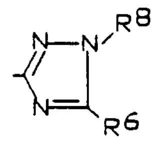 ,

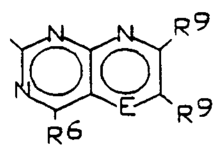 oder 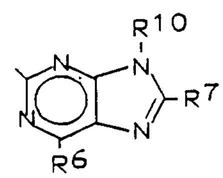

| | |
|---|---|
| E | CH oder N, |
| G | $CH_2$ oder O, |
| $R^6$ | unabhängig voneinander H, Halogen, $(C_1$-$C_6)$Alkyl, $(C_1$-$C_6)$Alkoxy oder $(C_1$-$C_6)$-Alkylthio, wobei die vorgenannten Reste im Alkylteil ein- oder mehrfach durch Halogen oder ein- oder zweifach durch $(C_1$-$C_4)$Alkoxy oder $(C_1$-$C_4)$Alkylthio substituiert sein können; einen Rest $N(R^{11})_2$, $(C_3$-$C_6)$Cycloalkyl, $-OCHR^7COOR^{11}$, $(C_2$-$C_5)$Alkenyl, $(C_2$-$C_4)$Alkinyl, $(C_3$-$C_5)$Alkenyloxy oder $(C_3$-$C_5)$Alkinyloxy, |
| $R^7$ | H oder $(C_1$-$C_4)$Alkyl, |
| $R^8$ | $(C_1$-$C_4)$Alkyl, $-CHF_2$ oder $-CH_2CF_3$, |
| $R^9$ | unabhängig voneinander H, $(C_1$-$C_4)$Alkyl, $(C_1$-$C_4)$Alkoxy oder Halogen, |
| $R^{10}$ | H, $(C_1$-$C_4)$Alkyl, $-CHF_2$ oder $-CH_2CF_3$, |
| $R^{11}$ | unabhängig voneinander H, $(C_1$-$C_4)$Alkyl, $(C_2$-$C_4)$Alkenyl oder $(C_3$-$C_4)$Alkinyl, |
| $R^{12}$ | H, $(C_1$-$C_4)$Alkyl, $(C_1$-$C_4$-Alkoxy)carbonyl, $(C_1$-$C_4)$Alkoxymethyl, |
| X | O oder S und |
| Y | O, S, $CH_2$, NH oder $N(C_1$-$C_4)$Alkyl bedeuten. |

**2.** Verbindungen der Formel I von Anspruch 1, oder deren Salze worin
$R^1$ und $R^2$ = unabhängig voneinander H, $(C_1$-$C_4)$Alkyl oder $R^1$ und $R^2$ zusammen einen heterocyclischen Ring der Formel

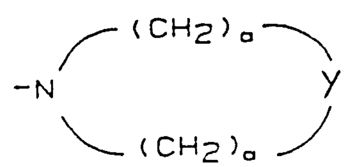

wobei

| | |
|---|---|
| $R^3$ | H, $(C_1$-$C_6)$Alkyl oder $(C_2$-$C_4)$Alkenyl, |
| $R^4$ | H, $(C_1$-$C_4)$Alkyl oder Allyl, |
| $R^5$ | einen Rest der Formel |

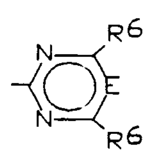

$R^6$ unabhängig voneinander Halogen, $(C_1$-$C_4)$Alkyl oder $(C_1$-$C_4)$Alkoxy, die beide halogeniert

sein können,

E CH oder N,

Y O und

a eine ganze Zahl von 1 bis 3 bedeuten.

**3.** Verbindungen der Formel I von Anspruch 1 oder 2, oder deren Salze, worin halogeniertes $(C_1-C_4)$Alkyl einen Rest der Gruppe $CF_3$, $-CH_2CH_2Cl$, $-CH_2CH_2Br$, $-CH_2CH_2CH_2Cl$, $-CF_2CF_2H$, $-CF_2CFClH$ oder $-CH_2CHFCF_3$ und halogeniertes $(C_1-C_4)$Alkoxy einen Rest der Gruppe $-OCHF_2$, $OCF_3$, $OCH_2CF_3$ oder $-OCH_2CH_2Cl$ bedeuten.

**4.** Verfahren zur Herstellung von Verbindungen der Formel I oder deren Salze von einem oder mehreren der Ansprüche 1 bis 3, dadurch gekennzeichnet, daß man

(a) eine Verbindung der Formel (II)

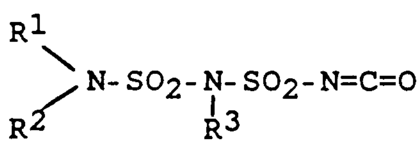 (II),

wobei $R^1$, $R^2$, $R^3$ die für Formel I angegebene Bedeutung außer H besitzen, mit einer Verbindung der Formel (III)

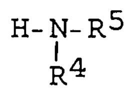 (III)

umsetzt, oder

(b) eine Verbindung der Formel (IV)

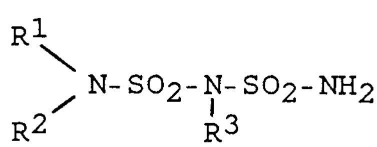 (IV)

wobei $R^1$, $R^2$, $R^3$ die für Formel I angegebene Bedeutung besitzen, mit einem (Thio)-Carbamat der Formel (V)

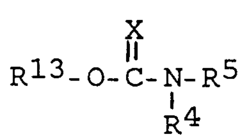 (V),

wobei $R^{13}$ = $(C_1-C_6)$Alkyl, $(C_1-C_4)$Halogenalkyl oder Phenyl, das unsubstituiert oder ein- oder mehrfach durch Halogen, $(C_1-C_4)$Alkyl oder $NO_2$ substituiert ist, umsetzt, oder

(c) eine Verbindung der Formel (VI)

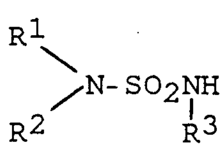 (VI)

wobei $R^1$, $R^2$, $R^3$ die für Formel I angegebene Bedeutung besitzen, mit einem Chlorsulfonylharnstoff der Formel (VII)

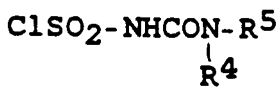

(VII)

wobei $R^4$ und $R^5$ die für Formel I angegebene Bedeutung besitzen, umsetzt, und die unter a), b) oder c) erhaltenen Verbindungen gegebenenfalls in ihre Salze überführt.

**5.** Herbizide oder pflanzenwachstumsregulierende Mittel, dadurch gekennzeichnet, daß sie eine Verbindung der Formel I von einem oder mehreren der Ansprüchen 1 bis 3 oder deren Salze neben inerten Trägerstoffen enthalten.

**6.** Verwendung von Verbindungen der Formel I oder deren Salze gemäß einem oder mehreren der Ansprüche 1 bis 3 als Herbizide oder Pflanzenwachstumsregulatoren.

**7.** Verfahren zur Bekämpfung von unerwünschten Pflanzen, dadurch gekennzeichnet, daß man auf diese oder deren Anbauflächen eine wirksame Menge einer Verbindung der Formel I oder deren Salze von einem oder mehreren der Ansprüche 1 bis 3 appliziert.

**8.** Verfahren zur Wachstumsregulierung von Pflanzen, dadurch gekennzeichnet, daß man auf diese oder die Anbauflächen eine wirksame Menge einer Verbindung der Formel I oder deren Salze von einem oder mehreren der Ansprüche 1 bis 3 appliziert.

**9.** Verbindungen der Formel (II)

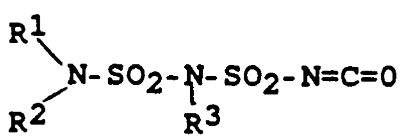

(II)

worin
$R^1$ , $R^2$ und $R^3$ die in Formel I nach einem oder mehreren der Ansprüche 1 bis 3 definierte Bedeutung außer Wasserstoff haben.

**10.** Verbindungen der Formel (IV)

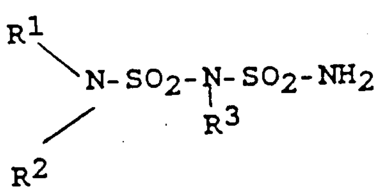

(IV)

worin $R^1$, $R^2$ und $R^3$ die für Formel (I) nach einem oder mehreren der Ansprüche 1 bis 3 definierte Bedeutung haben, ausgenommen die Verbindungen der Formel (IV), worin
a) $R^1$ = H, i-Propyl, Cyclohexyl oder Phenyl und $R^2$ = H sowie $R^3$ = H oder
b) $R^1$ = n-Butyl, $R^2$ = H und $R^3$ = n-Butyl oder
c) $R^1R^2N$ = N,N-Diethylamino oder Piperidin-1-yl bedeuten.

**Patentansprüche für folgenden Vertragsstaat : ES**

1. Verwendung von Verbindungen der Formel I oder deren Salze

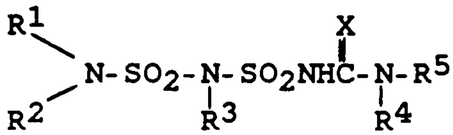

(I)

worin

$R^1$ und $R^2$ unabhängig voneinander H, $(C_1\text{-}C_8)$Alkyl, $(C_2\text{-}C_8)$Alkenyl, $(C_2\text{-}C_8)$Alkinyl, wobei diese Reste gegebenenfalls ein- oder mehrfach durch Halogen oder ein- oder zweifach durch $(C_1\text{-}C_4)$Alkoxy oder $(C_1\text{-}C_4)$Alkylthio substituiert sein können, $(C_1\text{-}C_4)$-Alkoxycarbonyl$(C_1\text{-}C_3)$alkyl, Phenyl, das unsubstituiert oder ein- oder mehrfach durch $(C_1\text{-}C_4)$Alkyl, $(C_1\text{-}C_4)$Alkoxy, $(C_1\text{-}C_4)$Alkylthio, $(C_1\text{-}C_4)$Alkylsulfinyl, $(C_1\text{-}C_4)$-Alkylsulfonyl, wobei die vorgenannten Reste im Alkylteil mehrfach durch Halogen oder ein- oder zweifach durch $(C_1\text{-}C_4)$Alkoxy substituiert sein können, $(C_1\text{-}C_4\text{-Alkoxy})$-carbonyl, Halogen oder $NO_2$, substituiert ist, oder $R^1$ und $R^2$ zusammen mit dem sie verknüpfenden N-Atom einen heterocyclischen Ring der Formel

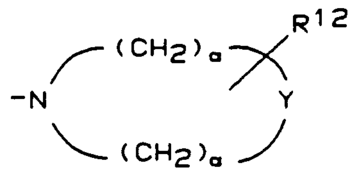

wobei a = unabhängig voneinander eine ganze Zahl von 1 bis 3, bedeutet,

$R^3$ H, $(C_1\text{-}C_{12})$Alkyl, $(C_2\text{-}C_8)$Alkenyl, $(C_2\text{-}C_8)$Alkinyl, wobei diese Reste ein- oder mehr ach durch Halogen oder ein- oder zweifach durch $(C_1\text{-}C_4)$Alkoxy oder $(C_1\text{-}C_4)$Alkylthio substituiert sein können, $(C_1\text{-}C_4\text{-Alkoxy})$carbonyl$(C_1\text{-}C_3)$alkyl, $-(CH_2)_b$Phenyl, wobei b = eine ganze Zahl von 0 bis 2 bedeutet und der Phenylrest unsubstituiert oder ein- oder mehrfach durch $(C_1\text{-}C_4)$Alkyl, $(C_1\text{-}C_4)$Alkoxy, $(C_1\text{-}C_4)$Alkylthio, $(C_1\text{-}C_4)$-Alkylsulfonyl, wobei die vorgenannten Reste im Alkylteil ein- oder mehrfach durch Halogen substituiert sein können, durch $(C_1\text{-}C_4)$Alkoxycarbonyl, Halogen oder $NO_2$ substituiert ist, oder $R^2$ und $R^3$ zusammen eine $(C_2\text{-}C_4)$Alkylenkette,

$R^4$ H, $(C_1\text{-}C_6)$Alkyl, $(C_2\text{-}C_8)$Alkenyl, $(C_2\text{-}C_8)$Alkinyl oder $(C_1\text{-}C_4)$Alkoxy,

$R^5$ einen Rest der Formel

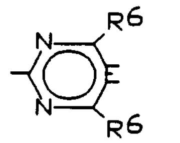 , 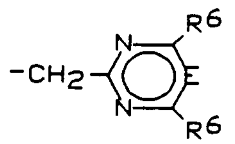 , 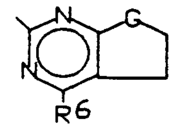 ,

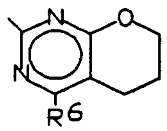 , 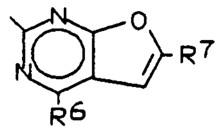 , 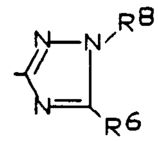 ,

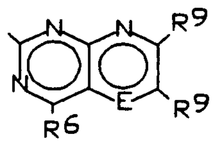 oder 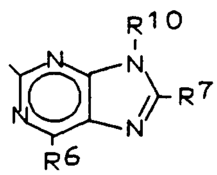

| | |
|---|---|
| E | CH oder N, |
| G | $CH_2$ oder O, |
| $R^6$ | unabhängig voneinander H, Halogen, $(C_1\text{-}C_6)$Alkyl, $(C_1\text{-}C_6)$Alkoxy oder $(C_1\text{-}C_6)$-Alkylthio, wobei die vorgenannten Reste im Alkylteil ein- oder mehrfach durch Halogen oder ein- oder zweifach durch $(C_1\text{-}C_4)$Alkoxy oder $(C_1\text{-}C_4)$Alkylthio substituiert sein können; einen Rest $N(R^{11})_2$, $(C_3\text{-}C_6)$Cycloalkyl, $-OCHR^7COOR^{11}$, $(C_2\text{-}C_5)$Alkenyl, $(C_2\text{-}C_4)$Alkinyl, $(C_3\text{-}C_5)$Alkenyloxy oder $(C_3\text{-}C_5)$Alkinyloxy, |
| $R^7$ | H oder $(C_1\text{-}C_4)$Alkyl, |
| $R^8$ | $(C_1\text{-}C_4)$Alkyl, $-CHF_2$ oder $-CH_2CF_3$, |
| $R^9$ | unabhängig voneinander H, $(C_1\text{-}C_4)$Alkyl, $(C_1\text{-}C_4)$Alkoxy oder Halogen, |
| $R^{10}$ | H, $(C_1\text{-}C_4)$Alkyl, $-CHF_2$ oder $-CH_2CF_3$, |
| $R^{11}$ | unabhängig voneinander H, $(C_1\text{-}C_4)$Alkyl, $(C_2\text{-}C_4)$Alkenyl oder $(C_3\text{-}C_4)$Alkinyl, |
| $R^{12}$ | H, $(C_1\text{-}C_4)$Alkyl, $(C_1\text{-}C_4\text{-Alkoxy})$carbonyl, $(C_1\text{-}C_4)$Alkoxymethyl, |
| X | O oder S und |
| Y | O, S, $CH_2$, NH oder $N(C_1\text{-}C_4)$Alkyl bedeuten, als Herbizide oder Pflanzenwachstumsregulatoren. |

**2.** Verwendung nach Anspruch 1, dadurch gekennzeichnet, daß in Formel (I) $R^1$ und $R^2$ = unabhängig voneinander H, $(C_1\text{-}C_4)$Alkyl oder $R^1$ und $R^2$ zusammen einen heterocyclischen Ring der Formel

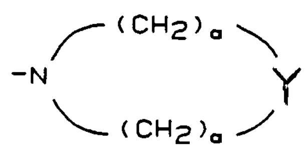

wobei

| | |
|---|---|
| $R^3$ | H, $(C_1\text{-}C_6)$Alkyl oder $(C_2\text{-}C_4)$Alkenyl, |
| $R^4$ | H, $(C_1\text{-}C_4)$Alkyl oder Allyl, |
| $R^5$ | einen Rest der Formel |

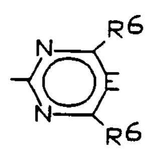

$R^6$ unabhängig voneinander Halogen, $(C_1\text{-}C_4)$Alkyl oder $(C_1\text{-}C_4)$Alkoxy, die beide halogeniert sein können,
E CH oder N,
Y O und
a zusammen eine ganze Zahl von 1 bis 3 bedeuten.

**3.** Verwendung nach Anspruch 1 oder 2, dadurch gekennzeichnet, daß in Formel I halogeniertes $(C_1\text{-}C_4)$-Alkyl einen Rest der Gruppe $CF_3$, $-CH_2CH_2Cl$, $-CH_2CH_2Br$, $-CH_2CH_2CH_2Cl$, $-CF_2CF_2H$, $-CF_2CFClH$ oder $-CH_2CHFCF_3$ und halogeniertes $(C_1\text{-}C_4)$Alkoxy einen Rest der Gruppe $-OCHF_2$, $OCF_3$, $OCH_2CF_3$ oder $-OCH_2CH_2Cl$ bedeuten.

**4.** Verfahren zur Herstellung von Verbindungen der Formel I oder deren Salze von einem oder mehreren der Ansprüchen 1 bis 3, dadurch gekennzeichnet, daß man
(a) eine Verbindung der Formel (II)

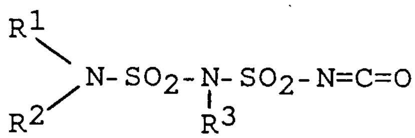
(II),

wobei $R^1$, $R^2$, $R^3$ die für Formel I angegebene Bedeutung außer H besitzen, mit einer Verbindung der Formel (III)

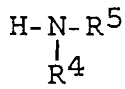
(III)

umsetzt, oder
(b) eine Verbindung der Formel (IV)

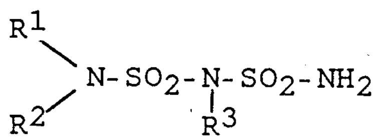
(IV)

wobei $R^1$, $R^2$, $R^3$ die für Formel I angegebene Bedeutung besitzen, mit einem (Thio)-Carbamat der Formel (V)

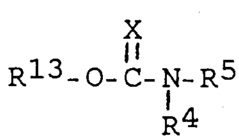
(V),

wobei $R^{13}$ = $(C_1\text{-}C_6)$Alkyl, $(C_1\text{-}C_4)$Halogenalkyl oder Phenyl, das unsubstituiert oder ein- oder mehrfach durch Halogen, $(C_1\text{-}C_4)$Alkyl oder $NO_2$ substituiert ist, umsetzt, oder

(c) eine Verbindung der Formel (VI)

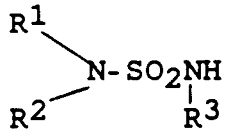

(VI)

wobei $R^1$, $R^2$, $R^3$ die für Formel I angegebene Bedeutung besitzen, mit einem Chlorsulfonylharnstoff der Formel (VII)

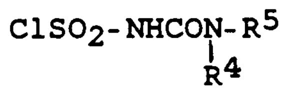

(VII)

wobei $R^4$ und $R^5$ die für Formel I angegebene Bedeutung besitzen, umsetzt, und die unter a), b) oder c) erhaltenen Verbindungen gegebenenfalls in ihre Salze überführt.

**5.** Verfahren zur Bekämpfung von unerwünschten Pflanzen, dadurch gekennzeichnet, daß man auf diese oder deren Anbauflächen eine wirksame Menge einer Verbindung der Formel I oder deren Salze von einem oder mehreren der Ansprüche 1 bis 3 appliziert.

**6.** Verfahren zur Wachstumsregulierung von Pflanzen, dadurch gekennzeichnet, daß man auf diese oder die Anbauflächen eine wirksame Menge einer Verbindung der Formel I oder deren Salze von einem oder mehreren der Ansprüche 1 bis 3 appliziert.

**Claims**

**Claims for the following Contracting States : AT, BE, CH, DE, FR, GB, IT, LI, NL**

**1.** A compound of the formula I or a salt thereof

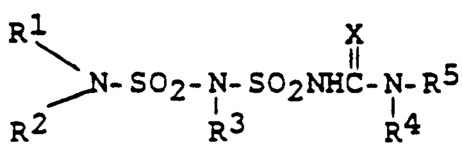

(I)

where

$R^1$ and $R^2$ independently of one another are H, or $(C_1\text{-}C_8)$alkyl, $(C_2\text{-}C_8)$alkenyl or $(C_2\text{-}C_8)$alkynyl, it being possible for these radicals optionally to be monosubstituted or polysubstituted by halogen or monosubstituted or disubstituted by $(C_1\text{-}C_4)$alkoxy or $(C_1\text{-}C_4)$alkylthio, or are $(C_1\text{-}C_4)$alkoxycarbonyl$(C_1\text{-}C_3)$alkyl, or phenyl which is unsubstituted or monosubstituted or polysub-stituted by $(C_1\text{-}C_4)$alkyl, $(C_1\text{-}C_4)$alkoxy, $(C_1\text{-}C_4)$alkylthio, $(C_1\text{-}C_4)$alkylsulfinyl or $(C_1\text{-}C_4)$alkylsulfonyl, it being possible for the abovementioned radicals to be polysubstituted in the alkyl moiety by halogen or monosubstituted or disubstituted in the alkyl moiety by $(C_1\text{-}C_4)$alkoxy, or phenyl which is unsubstituted or monosubstituted or polysubstituted by $(C_1\text{-}C_4\text{-alkoxy})$carbonyl, halogen or $NO_2$, or $R^1$ and $R^2$ together with the N atom by which they are linked are a heterocyclic ring of the formula

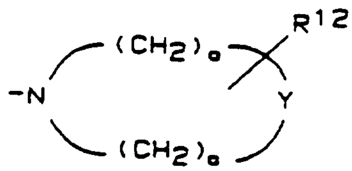,

a indices independently of one another being an integer from 1 to 3,

$R^3$ is H, $(C_1\text{-}C_{12})$alkyl, $(C_2\text{-}C_8)$alkenyl or $(C_2\text{-}C_8)$alkynyl, it being possible for these radicals to be monosubstituted or polysubstituted by halogen or monosubstituted or disubstituted by $(C_1\text{-}C_4)$alkoxy or $(C_1\text{-}C_4)$alkylthio, or $R^3$ is $(C_1\text{-}C_4$-alkoxy)carbonyl$(C_1\text{-}C_3)$alkyl, or $-(CH_2)_b$-phenyl, b being an integer from 0 to 2 and the phenyl radical being unsubstituted or monosubstituted or polysubstituted by $(C_1\text{-}C_4)$alkyl, $(C_1\text{-}C_4)$-alkoxy, $(C_1\text{-}C_4)$alkylthio or $(C_1\text{-}C_4)$alkylsulfonyl, it being possible for the abovementioned radicals to be monosubstituted or polysubstituted in the alkyl moiety by halogen, or phenyl being substituted by $(C_1\text{-}C_4)$alkoxycarbonyl, halogen or $NO_2$, or $R^2$ and $R^3$ together are a $(C_2\text{-}C_4)$alkylene chain,

$R^4$ is H, $(C_1\text{-}C_6)$alkyl, $(C_2\text{-}C_8)$alkenyl, $(C_2\text{-}C_8)$alkynyl or $(C_1\text{-}C_4)$alkoxy,

$R^5$ is a radical of the formula

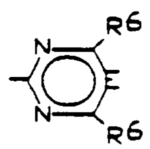 , 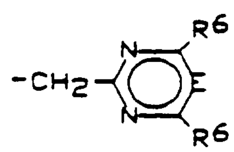 , 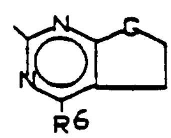 ,

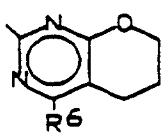 , 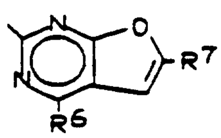 , 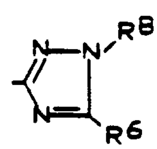 ,

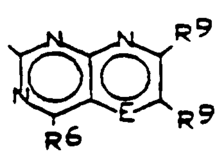 or 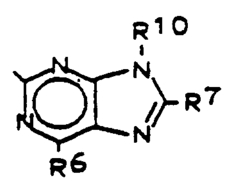

E is CH or N,

G is $CH_2$ or O,

$R^6$ radicals independently of one another are H, halogen, $(C_1\text{-}C_6)$alkyl, $(C_1\text{-}C_6)$alkoxy or $(C_1\text{-}C_6)$alkylthio, it being possible for the abovementioned radicals to be monosubstituted or polysubstituted in the alkyl moiety by halogen or monosubstituted or disubstituted in the alkyl moiety by $(C_1\text{-}C_4)$alkoxy or $(C_1\text{-}C_4)$alkylthio; or is a radical $N\text{-}(R^{11})_2$, $(C_3\text{-}C_6)$cycloalkyl, - $-OCHR^7COOR^{11}$, $(C_2\text{-}C_5)$alkenyl, $(C_2\text{-}C_4)$alkynyl, $(C_3\text{-}C_5)$-alkenyloxy or $(C_3\text{-}C_5)$alkynyloxy,

$R^7$ is H or $(C_1\text{-}C_4)$alkyl,

$R^8$ is $(C_1\text{-}C_4)$alkyl, $-CHF_2$ or $-CH_2CF_3$,

$R^9$ radicals independently of one another are H, $(C_1\text{-}C_4)$alkyl, $(C_1\text{-}C_4)$alkoxy or halogen,

$R^{10}$ is H, $(C_1\text{-}C_4)$alkyl, $-CHF_2$ or $-CH_2CF_3$,

$R^{11}$ radicals independently of one another are H, $(C_1\text{-}C_4)$alkyl, $(C_2\text{-}C_4)$alkenyl or $(C_3\text{-}C_4)$-alkynyl,

$R^{12}$ is H, $(C_1\text{-}C_4)$alkyl, $(C_1\text{-}C_4$alkoxy)carbonyl or $(C_1\text{-}C_4)$alkoxymethyl,

X is O or S and

Y is O, S, $CH_2$, NH or $N(C_1\text{-}C_4)$alkyl.

**2.** A compound of the formula I of claim 1 or a salt thereof where $R^1$ and $R^2$ independently of one another are H or $(C_1\text{-}C_4)$alkyl, or $R^1$ and $R^2$ together are a heterocyclic ring of the formula

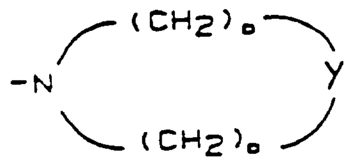

,

$R^3$ being H, $(C_1\text{-}C_6)$alkyl or $(C_2\text{-}C_4)$alkenyl,
$R^4$ being H, $(C_1\text{-}C_4)$alkyl or allyl,
$R^5$ being a radical of the formula

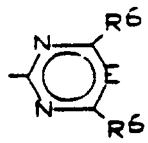

,

$R^6$ radicals independently of one another being halogen or $(C_1\text{-}C_4)$alkyl or $(C_1\text{-}C_4)$alkoxy, it being possible for these two to be halogenated,
E being CH or N,
Y being O and
a indices being an integer from 1 to 3.

**3.** A compound of the formula I of claim 1 or 2, or a salt thereof, where halogenated $(C_1\text{-}C_4)$alkyl is a radical of the group $CF_3$, $-CH_2CH_2Cl$, $-CH_2CH_2Br$, $-CH_2CH_2CH_2Cl$, $-CF_2CF_2H$, $-CF_2CFClH$ or $-CH_2CHFCF_3$, and halogenated $(C_1\text{-}C_4)$alkoxy is a radical of the group $-OCHF_2$, $OCF_3$, $OCH_2CF_3$ or $-OCH_2CH_2Cl$.

**4.** A process for the preparation of a compound of the formula I or a salt thereof, of one or more of claims 1 to 3, which comprises
(a) reacting a compound of the formula (II)

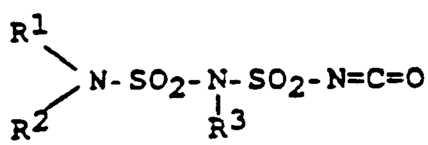

(II),

$R^1$, $R^2$ and $R^3$ having the meaning indicated in the case of formula I with the exception of H, with a compound of the formula (III)

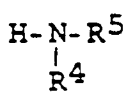

(III),

or

(b) reacting a compound of the formula (IV)

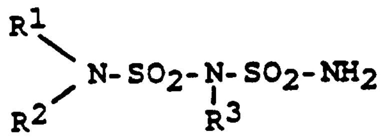

(IV),

$R^1$, $R^2$ and $R^3$ having the meaning indicated in the case of formula I, with a (thio)carbamate of the formula (V)

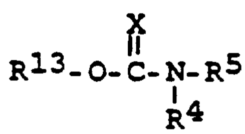

(V),

$R^{13}$ being $(C_1$-$C_6)$alkyl, $(C_1$-$C_4)$haloalkyl or phenyl which is unsubstituted or monosubstituted or polysubstituted by halogen, $(C_1$-$C_4)$alkyl or $NO_2$, or

(c) reacting a compound of the formula (VI)

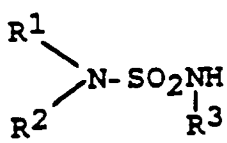

(VI),

$R^1$, $R^2$ and $R^3$ having the meaning indicated in the case of formula I, with a chlorosulfonylurea of the formula (VII)

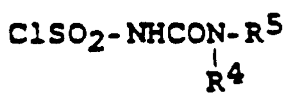

(VII),

$R^4$ and $R^5$ having the meaning indicated in the case of formula I, and, if desired, converting the compounds obtained by a), b) or c) into their salts.

**5.** A herbicidal or plant growth-regulating agent, containing a compound of the formula I of one or more of claims 1 to 3 or a salt thereof, besides inert carriers.

**6.** The use of a compound of the formula I or a salt thereof as claimed in one or more of claims 1 to 3, as a herbicide or plant growth regulator.

**7.** A method of controlling undesired plants, which comprises applying an effective amount of a compound of the formula I or a salt thereof, of one or more of claims 1 to 3, to these plants or the areas where they are grown.

**8.** A method of regulating plant growth, which comprises applying an effective amount of a compound of the formula I or a salt thereof, of one or more of claims 1 to 3, to these plants or the areas where they are grown.

**9.** A compound of the formula (II)

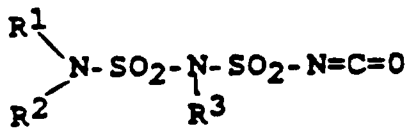

(II)

where

$R^1$, $R^2$ and $R^3$ have the meaning defined in formula I of one or more of claims 1 to 3, with the exception of hydrogen.

**10.** A compound of the formula (IV)

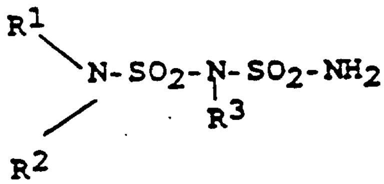

(IV)

where $R^1$, $R^2$ and $R^3$ have the meaning defined in the case of the formula (I) of one or more of claims 1 to 3, with the exception of a compound of the formula (IV) where

a) $R^1$ is H, isopropyl, cyclohexyl or phenyl and $R^2$ is H and $R^3$ is H, or

b) $R^1$ is n-butyl, $R^2$ is H and $R^3$ is n-butyl, or

c) $R^1R^2N$ is N,N-diethylamino or 1-piperidyl.

**Claims for the following Contracting State : ES**

**1.** The use of a compound of the formula I or a salt thereof

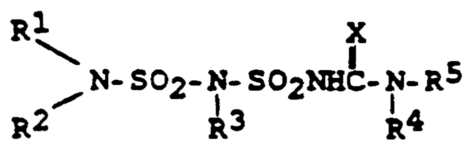

(I)

where

$R^1$ and $R^2$ independently of one another are H, or $(C_1\text{-}C_8)$alkyl, $(C_2\text{-}C_8)$alkenyl or $(C_2\text{-}C_8)$alkynyl, it being possible for these radicals optionally to be monosubstituted or polysubstituted by halogen or monosubstituted or disubstituted by $(C_1\text{-}C_4)$alkoxy or $(C_1\text{-}C_4)$alkylthio, or are $(C_1\text{-}C_4)$alkoxycarbonyl$(C_1\text{-}C_3)$alkyl, or phenyl which is unsubstituted or monosubstituted or polysubstituted by $(C_1\text{-}C_4)$alkyl, $(C_1\text{-}C_4)$alkoxy, $(C_1\text{-}C_4)$alkylthio, $(C_1\text{-}C_4)$alkylsulfinyl or $(C_1\text{-}C_4)$alkylsulfonyl, it being possible for the abovementioned radicals to be polysubstituted in the alkyl moiety by halogen or monosubstituted or disubstituted in the alkyl moiety by $(C_1\text{-}C_4)$ alkoxy, or phenyl which is unsubstituted or monosubstituted or polysubstituted by $(C_1\text{-}C_4\text{-alkoxy})$carbonyl, halogen or $NO_2$, or $R^1$ and $R^2$ together with the N atom by which they are linked are a heterocyclic ring of the formula

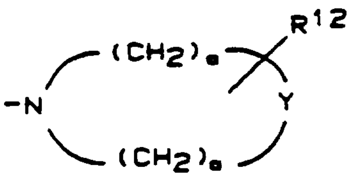 ,

a indices independently of one another being an integer from 1 to 3,

$R^3$ is H, $(C_1\text{-}C_{12})$alkyl, $(C_2\text{-}C_8)$alkenyl or $(C_2\text{-}C_8)$alkynyl, it being possible for these radicals to be monosubstituted or polysubstituted by halogen or monosubstituted or disubstituted by $(C_1\text{-}C_4)$alkoxy or $(C_1\text{-}C_4)$alkylthio, or $R^3$ is $(C_1\text{-}C_4$-alkoxy)carbonyl$(C_1\text{-}C_3)$alkyl, or - $(CH_2)_b$-phenyl, b being an integer from 0 to 2 and the phenyl radical being unsubstituted or monosubstituted or polysubstituted by $(C_1\text{-}C_4)$alkyl, $(C_1C_4)$-alkoxy, $(C_1\text{-}C_4)$alkylthio or $(C_1\text{-}C_4)$alkylsulfonyl, it being possible for the abovementioned radicals to be monosubstituted or polysubstituted in the alkyl moiety by halogen, or phenyl being substituted by $(C_1\text{-}C_4)$alkoxycarbonyl, halogen or $NO_2$, or $R^2$ and $R^3$ together are a $(C_2\text{-}C_4)$alkylene chain,

$R^4$ is H, $(C_1\text{-}C_6)$alkyl, $(C_2\text{-}C_8)$alkenyl, $(C_2\text{-}C_8)$alkynyl or $(C_1\text{-}C_4)$alkoxy,

$R^5$ is a radical of the formula

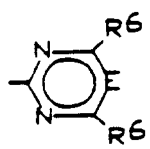 , 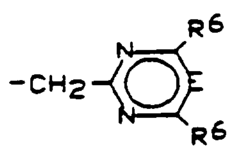 , 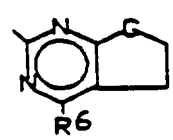 ,

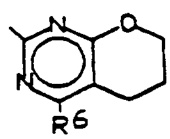 , 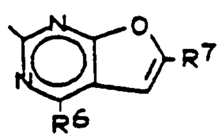 , 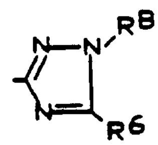 ,

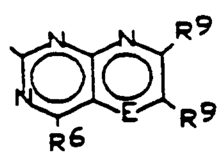 or 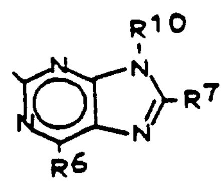

E is CH or N,

G is $CH_2$ or O,

$R^6$ radicals independently of one another are H, halogen, $(C_1\text{-}C_6)$alkyl, $(C_1\text{-}C_6)$alkoxy or $(C_1\text{-}C_6)$alkylthio, it being possible for the abovementioned radicals to be monosubstituted or polysubstituted in the alkyl moiety by halogen or monosubstituted or disubstituted in the alkyl moiety by $(C_1\text{-}C_4)$alkoxy or $(C_1\text{-}C_4)$alkylthio; or is a radical N-$(R^{11})_2$, $(C_3\text{-}C_6)$cycloalkyl, $-OCHR^7COOR^{11}$, $(C_2\text{-}C_5)$alkenyl, $(C_2\text{-}C_4)$alkynyl, $(C_3\text{-}C_5)$-alkenyloxy or $(C_3\text{-}C_5)$alkynyloxy,

$R^7$ is H or $(C_1\text{-}C_4)$alkyl,

$R^8$ is $(C_1\text{-}C_4)$alkyl, $-CHF_2$ or $-CH_2CF_3$,

$R^9$ radicals independently of one another are H, $(C_1\text{-}C_4)$alkyl, $(C_1\text{-}C_4)$alkoxy or halogen,

$R^{10}$ is H, $(C_1\text{-}C_4)$alkyl, $-CHF_2$ or $-CH_2CF_3$,

$R^{11}$ radicals independently of one another are H, $(C_1\text{-}C_4)$alkyl, $(C_2\text{-}C_4)$alkenyl or $(C_3\text{-}C_4)$-alkynyl,

$R^{12}$ is H, $(C_1\text{-}C_4)$alkyl, $(C_1\text{-}C_4$alkoxy)carbonyl or $(C_1\text{-}C_4)$alkoxymethyl,

X is O or S and

Y is O, S, $CH_2$, NH or N$(C_1\text{-}C_4)$alkyl,

as a herbicide or plant growth regulator.

**2.** The use as claimed in claim 1, wherein in formula (I) $R^1$ and $R^2$ independently of one another are H or $(C_1-C_4)$alkyl, or $R^1$ and $R^2$ together are a heterocyclic ring of the formula

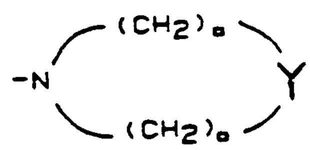

,

$R^3$ being H, $(C_1-C_6)$alkyl or $(C_2-C_4)$alkenyl,
$R^4$ being H, $(C_1-C_4)$alkyl or allyl,
$R^5$ being a radical of the formula

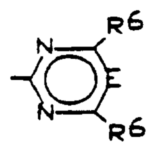

,

$R^6$ radicals independently of one another being halogen or $(C_1-C_4)$alkyl or $(C_1-C_4)$alkoxy, it being possible for these two to be halogenated,
E being CH or N,
Y being O and
a indices being an integer from 1 to 3.

**3.** The use as claimed in claim 1 or 2, wherein in formula I halogenated $(C_1-C_4)$alkyl is a radical of the group $CF_3$, $-CH_2CH_2Cl$, $-CH_2CH_2Br$, $-CH_2CH_2CH_2Cl$, $-CF_2CF_2H$, $-CF_2CFClH$ or $-CH_2CHFCF_3$, and halogenated $(C_1-C_4)$alkoxy is a radical of the group $-OCHF_2$, $OCF_3$, $OCH_2CF_3$ or $-OCH_2CH_2Cl$.

**4.** A process for the preparation of a compound of the formula I or a salt thereof, of one or more of claims 1 to 3, which comprises
(a) reacting a compound of the formula (II)

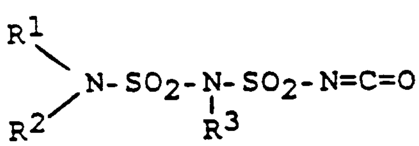

(II),

$R^1$, $R^2$ and $R^3$ having the meaning indicated in the case of formula I with the exception of H, with a compound of the formula (III)

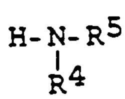

(III),

or

(b) reacting a compound of the formula (IV)

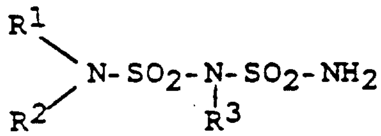

(IV),

$R^1$, $R^2$ and $R^3$ having the meaning indicated in the case of formula I, with a (thio)carbamate of the formula (V)

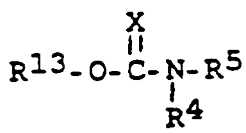

(V),

$R^{13}$ being $(C_1\text{-}C_6)$alkyl, $(C_1\text{-}C_4)$haloalkyl or phenyl which is unsubstituted or monosubstituted or polysubstituted by halogen, $(C_1\text{-}C_4)$alkyl or $NO_2$, or

(c) reacting a compound of the formula (VI)

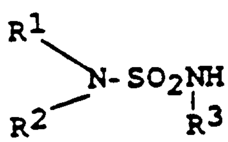

(VI),

$R^1$, $R^2$ and $R^3$ having the meaning indicated in the case of formula I, with a chlorosulfonylurea of the formula (VII)

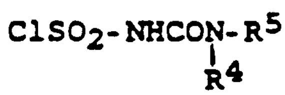

(VII),

$R^4$ and $R^5$ having the meaning indicated in the case of formula I, and, if desired, converting the compounds obtained by a), b) or c) into their salts.

**5.** A method of controlling undesired plants, which comprises applying an effective amount of a compound of the formula I or a salt thereof, of one or more of claims 1 to 3, to these plants or the areas where they are grown.

**6.** A method of regulating plant growth, which comprises applying an effective amount of a compound of the formula I or a salt thereof, of one or more of claims 1 to 3, to these plants or the areas where they are grown.

**Revendications**

**Revendications pour les Etats contractants suivants : AT, BE, CH, DE, FR, GB, IT, LI, NL**

**1.** Composés de formule I ci-dessous et sels de ces composés

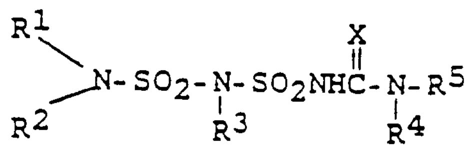

(I)

dans laquelle

$R^1$ et $R^2$ représentent chacun, indépendamment l'un de l'autre, l'hydrogène, un $(C_1-C_8)$alkyle, $(C_2-C_8)$alcényle ou $(C_2-C_8)$alcynyle qui peuvent éventuellement avoir comme substituants un ou plusieurs atomes d'halogènes ou un ou deux groupes $(C_1-C_4)$alcoxy ou $(C_1-C_4)$alkylthio, un $(C_1-C_4$-alcoxy)carbonyl$(C_1-C_3)$alkyle, un phényle sans substituants ou bien avec un ou plusieurs groupes $(C_1-C_4)$alkyles, $(C_1-C_4)$alcoxy, $(C_1-C_4)$alkylthio, $(C_1-C_4)$alkylsulfinyle ou $(C_1-C_4)$alkylsulfonyles, groupes qui peuvent avoir eux-mêmes comme substituants sur la partie alkylique plusieurs atomes d'halogènes, ou bien un ou deux $(C_1-C_4)$alcoxy, $(C_1-C_4$-alcoxy)carbonyles, halogènes ou $NO_2$, ou bien $R^1$ et $R^2$ forment ensemble et avec l'atome d'azote qui les relie un hétérocycle de formule

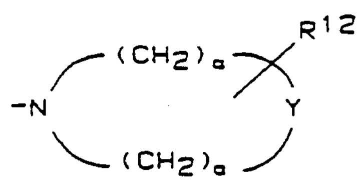

a étant un entier de 1 à 3, indépendamment l'un de l'autre,

$R^3$ représente H, un $(C_1-C_{12})$alkyle, $(C_2-C_8)$alcényle ou $(C_2-C_8)$alcynyle, qui peuvent avoir chacun comme substituants un ou plusieurs atomes d'halogènes ou un ou deux groupes $(C_1-C_4)$alcoxy ou $(C_1-C_4)$alkylthio, un $(C_1-C_4$-alcoxy)carbonyl$(C_1-C_3)$alkyle, un $-(CH_2)_b$-phényle, b étant un entier de 0 à 2 et le phényle étant sans substituants ou avec un ou plusieurs substituants $(C_1-C_4)$alkyles, $(C_1-C_4)$alcoxy, $(C_1-C_4)$alkylthio ou $(C_1-C_4)$alkylsulfonyles, qui peuvent avoir eux-mêmes sur la partie alkylique un ou plusieurs halogènes, ou encore $(C_1-C_4)$alcoxycarbonyles, halogènes ou $NO_2$, ou bien $R^2$ et $R^3$ forment ensemble une chaîne alkylène en $C_2-C_4$,

$R^4$ représente H, un $(C_1-C_6)$alkyle, un $(C_2-C_8)$alcényle, $(C_2-C_8)$alcynyle ou $(C_1-C_4)$alcoxy,

$R^5$ un radical de formule

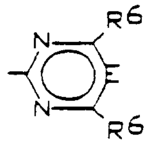 , 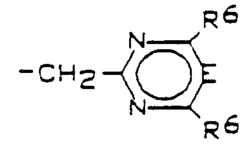 , 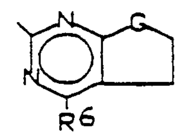 ,

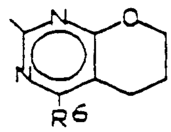 , 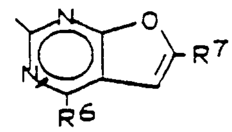 , 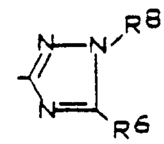 ,

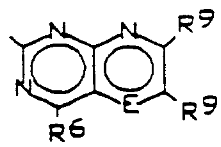 ou 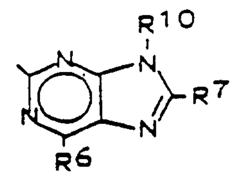

| | |
|---|---|
| E | désignant CH ou N, |
| G | $CH_2$ ou O, |
| $R^6$ | indépendamment l'un de l'autre, H, un halogène, un $(C_1\text{-}C_6)$alkyle, $(C_1\text{-}C_6)$alcoxy ou $(C_1\text{-}C_6)$alkylthio, qui peuvent avoir sur la partie alkylique un ou plusieurs atomes d'halogènes ou un ou deux groupes $(C_1\text{-}C_4)$-alcoxy ou $(C_1\text{-}C_4)$alkylthio ; ou encore un radical $N(R_{11})_2$, $(C_3\text{-}C_6)$cycloalkyle, $-OCHR^7COOR^{11}$, $(C_2\text{-}C_5)$alcényle, $(C_2\text{-}C_4)$alcynyle, $(C_3\text{-}C_5)$alcényloxy ou $(C_3\text{-}C_5)$alcynyloxy. |
| $R^7$ | H ou $(C_1\text{-}C_4)$alkyle, |
| $R^8$ | un $(C_1\text{-}C_4)$alkyle, $-CF_2$ ou $CH_2CF_3$, |
| $R^9$ | indépendamment l'un de l'autre, H, un $(C_1\text{-}C_4)$alkyle, $(C_1\text{-}C_4)$alcoxy ou un halogène, |
| $R^{10}$ | H, un $(C_1\text{-}C_4)$alkyle, $-CF_2$ ou $CH_2CF_3$, |
| $R^{11}$ | indépendamment l'un de l'autre, H, un $(C_1\text{-}C_4)$alcoxy, $(C_2\text{-}C_4)$alcényle ou $(C_3\text{-}C_4)$-alcynyle, |
| $R^{12}$ | H, un $(C_1\text{-}C_4)$alkyle, un $(C_1\text{-}C_4\text{-alcoxy})$carbonyle ou un $(C_1\text{-}C_4)$alcoxyméthyle, et |
| X | représente l'oxygène ou le soufre et |
| Y | l'oxygène; le soufre ou un groupe CH2, NH ou $N(C_1\text{-}C_4)$alkyle. |

**2.** Composés de formule I selon la revendication 1 et leurs sels, dans lesquels $R^1$ et $R^2$, indépendamment l'un de l'autre, sont chacun l'hydrogène ou un alkyle en $C_1\text{-}C_4$, ou bien forment ensemble un hétérocycle de formule

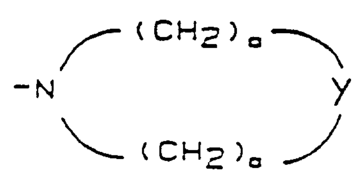

| | |
|---|---|
| $R^3$ | est l'hydrogène, un alkyle en $C_1\text{-}C_6$, ou un alcényle en $C_2\text{-}C_4$, |
| $R^4$ | l'hydrogène, un alkyle en $C_1\text{-}C_4$, ou un allyle, |
| $R^5$ | un radical de formule |

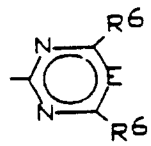

- $R^6$ indépendamment l'un de l'autre, un halogène un ($C_1$-$C_4$)alkyle ou un ($C_1$-$C_4$)alcoxy, qui peuvent être tous deux halogénés,
- E désigne CH ou N,
- Y l'oxygène et
- a un entier de 1 à 3.

**3.** Composés de formule I selon la revendication 1 ou 2 et sels de ces composés, dans lesquels l'alkyle en $C_1$-$C_4$ halogéné est un groupe $-CF_3$, $-CH_2CH_2Cl$, $-CH_2CH_2Br$, $-CH_2CH_2CH_2Cl$, $-CF_2CF_2H$, $-CF_2CFClH$, $-CH_2CHFCF_3$ et l'alcoxy en $C_1$-$C_4$ halogéné est un groupe $-OCHF_2$, $OCF_3$, $OCH_2CF_3$ ou $-OCH_2CH_2Cl$.

**4.** Procédés de préparation des composés de formule I ou de leurs sels selon une ou plusieurs des revendications 1 à 3, procédés caractérisés en ce que :

(a) on fait réagir un composé de formule II

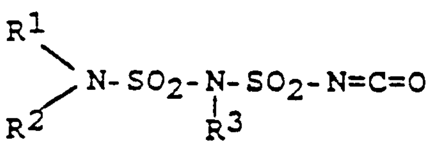 (II),

$R^1$, $R^2$ et $R^3$ ayant les mêmes significations que dans la formule I à l'exception de l'hydrogène, avec un composé de formule III

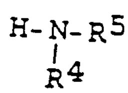 (III)

ou bien

(b) on fait réagir un composé de formule IV

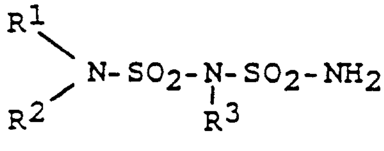 (IV)

$R^1$, $R^2$ et $R^3$ ayant les mêmes significations que dans la formule I, avec un (thio)carbamate de formule V

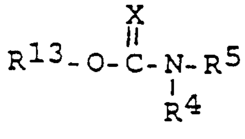 (V),

dans laquelle $R^{13}$ désigne un alkyle en $C_1$-$C_6$, un halogénoalkyle en $C_1$-$C_4$ ou un phényle sans substituants ou avec un ou plusieurs substituants pris parmi des halogènes, des alkyles en $C_1$-$C_4$ et le groupe $NO_2$, ou bien

(c) on fait réagir un composé de formule VI,

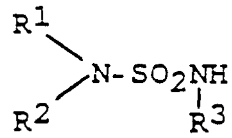 (VI)

$R^1$, $R^2$ et $R^3$ ayant les mêmes significations que dans la formule I, avec une chlorosulfonylurée de formule VII

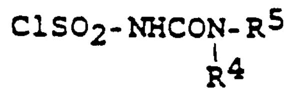 (VII)

$R^4$ et $R^5$ ayant les mêmes significations que dans la formule I, et le cas échéant on transforme en sels les composés obtenus en a), b) ou c).

**5.** Produits herbicides ou régulateurs de la croissance de végétaux, caractérisés en ce qu'ils comprennent un composé de formule I selon une ou plusieurs des revendications 1 à 3 ou un sel de ces composés, avec des matières inertes servant de supports.

**6.** L'emploi de composés de formule I ou de leurs sels selon une ou plusieurs des revendications 1 à 3 comme herbicides ou régulateurs de la croissance de plantes.

**7.** Procédé de lutte contre des plantes indésirables, procédé caractérisé en ce que l'on applique sur ces plantes ou sur leur surface de culture une quantité appropriée d'un composé de formule I ou d'un sel de ces composés selon une ou plusieurs des revendications 1 à 3.

**8.** Procédé de régulation de la croissance de plantes, procédé caractérisé en ce que l'on applique sur ces plantes ou sur leur surface de culture une quantité appropriée d'un composé de formule I ou d'un sel de ces composés selon une ou plusieurs des revendications 1 à 3.

**9.** Composés de formule II

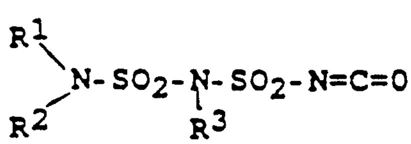 (II)

dans laquelle $R^1$, $R^2$ et $R^3$ ont les significations qui ont été indiquées pour la formule I à l'une des plusieurs des revendications 1 à 3, à l'exception de l'hydrogène.

**10.** Composés de formule IV

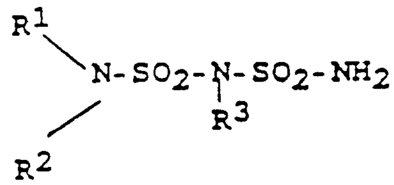

(IV)

dans laquelle $R^1$, $R^2$ et $R^3$ ont les significations qui ont été indiquées pour la formule I à l'une des plusieurs des revendications 1 à 3, à l'exception des composés dans lesquels

a) $R^1$ est l'hydrogène ou le groupe i-propyle, cyclohexyle ou phényle et $R^2$ et $R^3$ sont l'hydrogène, ou bien

b) $R^1$ est le groupe n-butyle, $R^2$ l'hydrogène et $R^3$ le groupe n-butyle, ou bien

c) $R^1R^2N$ est un groupe N,N-diéthylamino ou pipéridine-1-yle.

**Revendications pour l'Etat contractant suivant : ES**

**1.** L'emploi comme herbicides ou régulateurs de la croissance de plantes de composés de formule I ou de sels de ces composés

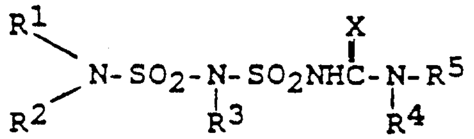

(I)

dans laquelle

$R^1$ et $R^2$ représentent chacun, indépendamment l'un de l'autre, l'hydrogène, un $(C_1-C_8)$alkyle, $(C_2-C_8)$alcényle ou $(C_2-C_8)$alcynyle qui peuvent éventuellement avoir comme substituants un ou plusieurs atomes d'halogènes ou un ou deux groupes $(C_1-C_4)$alcoxy ou $(C_1-C_4)$alkylthio, un $(C_1-C_4$-alcoxy)carbonyl$(C_1-C_3)$alkyle, un phényle sans substituants ou bien avec un ou plusieurs groupes $(C_1-C_4)$alkyles, $(C_1-C_4)$alcoxy, $(C_1-C_4)$alkylthio, $(C_1-C_4)$alkylsulfinyle ou $(C_1-C_4)$alkylsulfonyles, groupes qui peuvent avoir eux-mêmes comme substituants sur la partie alkylique plusieurs atomes d'halogènes, ou bien un ou deux $(C_1-C_4)$alcoxy, $(C_1-C_4$-alcoxy)carbonyles, halogènes ou $NO_2$, ou bien $R^1$ et $R^2$ forment ensemble et avec l'atome d'azote qui les relie un hétérocycle de formule

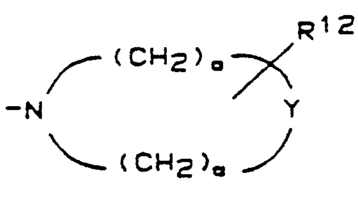

a étant un entier de 1 à 3, indépendamment l'un de l'autre,

$R^3$ représente H, un $(C_1-C_{12})$alkyle, $(C_2-C_8)$alcényle ou $(C_2-C_8)$alcynyle, qui peuvent avoir chacun comme substituants un ou plusieurs atomes d'halogènes ou un ou deux groupes $(C_1-C_4)$alcoxy ou $(C_1-C_4)$alkylthio, un $(C_1-C_4$-alcoxy)carbonyl$(C_1-C_3)$alkyle, un $-(CH_2)_b$-phényle, b étant un entier de 0 à 2 et le phényle étant sans substituants ou avec un ou plusieurs substituants $(C_1-C_4)$alkyles, $(C_1-C_4)$alcoxy, $(C_1-C_4)$alkylthio ou $(C_1-C_4)$alkylsulfonyles, qui peuvent avoir eux-mêmes sur la partie alkylique un ou plusieurs halogènes, ou encore $(C_1-C_4)$alcoxycarbonyles, halogènes ou $NO_2$, ou bien $R^2$ et $R^3$

forment ensemble une chaîne alkylène en $C_2$-$C_4$,

$R^4$ représente H, un ($C_1$-$C_6$)alkyle, un ($C_2$-$C_8$)alcényle, ($C_2$-$C_8$)alcynyle ou ($C_1$-$C_4$)alcoxy,

$R^5$ un radical de formule

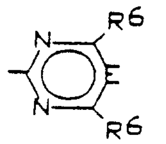 , 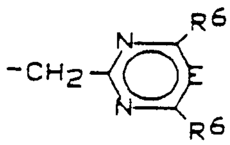 , 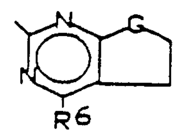 ,

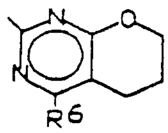 , 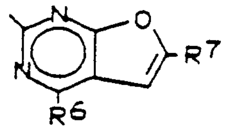 , 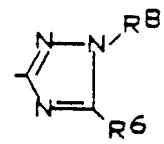 ,

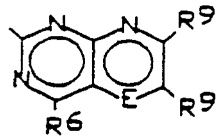 ou 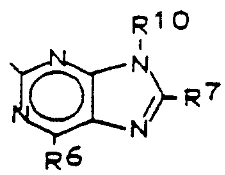

E désignant CH ou N,

G $CH_2$ ou O,

$R^6$ indépendamment l'un de l'autre, H, un halogène, un ($C_1$-$C_6$)alkyle, ($C_1$-$C_6$)alcoxy ou ($C_1$-$C_6$)alkylthio, qui peuvent avoir sur la partie alkylique un ou plusieurs atomes d'halogènes ou un ou deux groupes ($C_1$-$C_4$)-alcoxy ou ($C_1$-$C_4$)alkylthio ; ou encore un radical $N(R_{11})_2$, ($C_3$-$C_6$)cycloalkyle, -$OCHR^7COOR^{11}$, ($C_2$-$C_5$)alcényle, ($C_2$-$C_4$)alcynyle, ($C_3$-$C_5$)alcényloxy ou ($C_3$-$C_5$)alcynyloxy.

$R^7$ H ou ($C_1$-$C_4$)alkyle,

$R^8$ un ($C_1$-$C_4$)alkyle, -$CF_2$ ou $CH_2CF_3$,

$R^9$ indépendamment l'un de l'autre, H, un ($C_1$-$C_4$)alkyle, ($C_1$-$C_4$)alcoxy ou un halogène,

$R^{10}$ H, un ($C_1$-$C_4$)alkyle, -$CF_2$ ou $CH_2CF_3$,

$R^{11}$ indépendamment l'un de l'autre, H, un ($C_1$-$C_4$)alcoxy, ($C_2$-$C_4$)alcényle ou ($C_3$-$C_4$)-alcynyle,

$R^{12}$ H, un ($C_1$-$C_4$)alkyle, un ($C_1$-$C_4$-alcoxy)carbonyle ou un ($C_1$-$C_4$)alcoxyméthyle, et

X représente l'oxygène ou le soufre et

Y l'oxygène, le soufre ou un groupe CH2, NH ou N($C_1$-$C_4$)alkyle.

**2.** Emploi selon la revendication 1 caractérisé en ce que dans la formule I
$R^1$ et $R^2$, indépendamment l'un de l'autre, sont chacun l'hydrogène ou un alkyle en $C_1$-$C_4$, ou bien forment ensemble un hétérocycle de formule

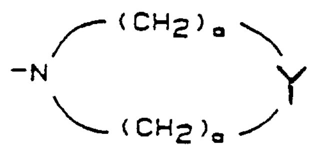

$R^3$ est l'hydrogène, un alkyle en $C_1$-$C_6$, ou un alcényle en $C_2$-$C_4$,

$R^4$ l'hydrogène, un alkyle en $C_1$-$C_4$, ou un allyle,

$R^5$ un radical de formule

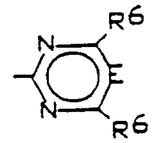

$R^6$ indépendamment l'un de l'autre, un halogène un ($C_1$-$C_4$)alkyle ou un ($C_1$-$C_4$)alcoxy, qui peuvent être tous deux halogénés,
E désigne CH ou N,
Y l'oxygène et
a un entier de 1 à 3.

**3.** Emploi selon la revendication 1 ou 2 caractérisé en ce que dans la formule I l'alkyle en $C_1$-$C_4$ halogéné est un groupe $-CF_3$, $-CH_2CH_2Cl$, $-CH_2CH_2Br$, $-CH_2CH_2CH_2Cl$, $-CF_2CF_2H$, $-CF_2CFClH$, $-CH_2CHFCF_3$ et l'alcoxy en $C_1$-$C_4$ halogéné est un groupe $-OCHF_2$, $OCF_3$, $OCH_2CF_3$ ou $-OCH_2CH_2Cl$.

**4.** Procédés de préparation des composés de formule I ou de leurs sels selon une ou plusieurs des revendications 1 à 3, procédés caractérisés en ce que :
(a) on fait réagir un composé de formule II

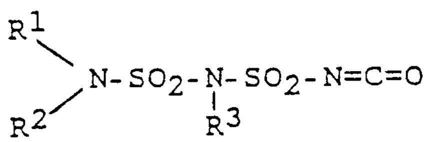 (II),

$R^1$, $R^2$ et $R^3$ ayant les mêmes significations que dans la formule I à l'exception de l'hydrogène, avec un composé de formule III

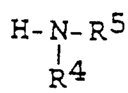 (III)

ou bien
(b) on fait réagir un composé de formule IV

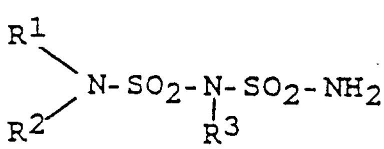 (IV)

$R^1$, $R^2$ et $R^3$ ayant les mêmes significations que dans la formule I, avec un (thio)carbamate de formule V

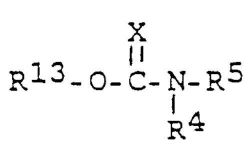 (V),

dans laquelle $R^{13}$ désigne un alkyle en $C_1$-$C_6$, un halogénoalkyle en $C_1$-$C_4$ ou un phényle sans substituants ou avec un ou plusieurs substituants pris parmi des halogènes, des alkyles en $C_1$-$C_4$ et le groupe $NO_2$, ou bien
(c) on fait réagir un composé de formule VI,

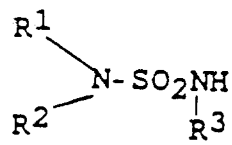

(VI)

$R^1$, $R^2$ et $R^3$ ayant les mêmes significations que dans la formule I, avec une chlorosulfonylurée de formule VII

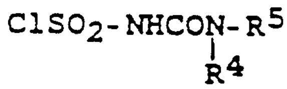

(VII)

$R^4$ et $R^5$ ayant les mêmes significations que dans la formule I, et le cas échéant on transforme en sels les composés obtenus en a), b) ou c).

**5.** Procédé de lutte contre des plantes indésirables, procédé caractérisé en ce que l'on applique sur ces plantes ou sur leur surface de culture une quantité appropriée d'un composé de formule I ou d'un sel de ces composés selon une ou plusieurs des revendications 1 à 3.

**6.** Procédé de régulation de la croissance de plantes, procédé caractérisé en ce que l'on applique sur ces plantes ou sur leur surface de culture une quantité appropriée d'un composé de formule I ou d'un sel de ces composés selon une ou plusieurs des revendications 1 à 3.